(19) Europäisches Patentamt
European Patent Office
Office européen des brevets

(11) Veröffentlichungsnummer: **0 085 995**
**B1**

(12) **EUROPÄISCHE PATENTSCHRIFT**

(45) Veröffentlichungstag der Patentschrift: **09.04.86**

(51) Int. Cl.⁴: **C 07 C 121/64**, C 07 C 121/75, C 09 K 19/32

(21) Anmeldenummer: **83200021.0**

(22) Anmeldetag: **10.01.83**

(54) **Anisotrope Verbindungen und Flüssigkristallmischungen.**

(30) Priorität: **04.02.82 CH 675/82**

(43) Veröffentlichungstag der Anmeldung: **17.08.83 Patentblatt 83/33**

(45) Bekanntmachung des Hinweises auf die Patenterteilung: **09.04.86 Patentblatt 86/15**

(84) Benannte Vertragsstaaten: **CH DE FR GB LI**

(56) Entgegenhaltungen:
**DE-A-2 931 637**
**DE-A-2 939 782**
**GB-A-2 027 027**
**GB-A-2 093 057**

(73) Patentinhaber: **Merck Patent Gesellschaft mit beschränkter Haftung, Frankfurter Strasse 250, D-6100 Darmstadt (DE)**

(72) Erfinder: **Huynh-Ba, Tuong, Dr., Stockmattstrasse 37, CH-5400 Baden (CH)**
Erfinder: **Kelly, Stephen Malcolm, Dr., Badenerstrasse 37, CH-5452 Oberrohrdorf (CH)**
Erfinder: **Osman, Maged A., Dr., Lerchenrain 1, CH-8046 Zürich (CH)**

**EP 0 085 995 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue anisotrope Verbindungen mit negativer DK-Anisotropie und Flüssigkristallmischungen (FK-Mischungen), welche diese Verbindungen als Komponenten enthalten.

Bekanntlich werden für verschiedene Typen von elektrooptischen Anzeigen, z. B. für die sogenannten ""Guest/Host-Displays" (GHD) und die homeotrop-nematischen Displays (HND), nematische FK-Mischungen mit einem stark negativen Wert von $\Delta\varepsilon$ benötigt (d. h. einem Negativwert von mindestens 2), wobei $\Delta\varepsilon$ als DK-Anisotropie oder DKA bezeichnet wird.

Die FK-Mischungen bzw. deren Komponenten müssen aber ausser einer negativen DKA auch eine Reihe von weiteren Eigenschaften erfüllen, wozu insbesondere eine hohe Stabilität in fotochemischer, allgemein chemischer und elektrochemischer Hinsicht sowie eine möglichst geringe Viskosität, geringe optische Anisotropie (geringe Doppelbrechungsneigung) und dergleichen Eigenschaften gehören.

Die bekannten Verbindungen mit negativer DKA werden diesen Forderungen nur in unzureichendem Masse gerecht; so haben die von J. C. Dubois et al in Mol. Cryst. Liqu. Cryst. 42 (1977) 139 sowie die in den DE-OS 28 35 662, 28 36 086, 26 13 293 und 22 40 864 und in SU-PS 562'547 beschriebenen Verbindungen eine aus zwei oder drei aromatischen Ringen aufgebaute Esterstruktur mit 1 bis 4 lateral gebundenen Gruppen, wie Nitrilgruppen; diese Verbindungen haben vergleichsweise hohe Werte der Viskosität und der optischen Anisotropie und zum Teil ungenügend stark negative DKA-Werte.

Zur Verminderung dieser Nachteile sind gemäss EP-OS 0023728 der Anmelderin bzw. gemäss GB-OS 2'027'708 sowie DE-OS 29 31 637 anisotrope Verbindungen mit negativer DKA bekannt bzw. vorgeschlagen worden, bei welchen durch Verwendung von trans-Cyclohexanringen oder durch Verwendung anderer Brückengruppen als Carboxylgruppen in der Grundstruktur die Viskosität der Verbindungen mit negativer DKA vermindert und die negative DKA durch Verwendung von zwei lateralen Nitrilgruppen an einem Phenylring der Struktur gesteuert werden kann, wobei der Klärpunkt durch diese lateralen Substituenten gesenkt wird.

Die einzigen zurzeit kommerziell erhältlichen nematischen FK-Mischungen mit relativ stark negativer DKA ($\Delta\varepsilon \approx -5$) sind die Produkte mit den Typenbezeichnungen ""EN 18" bzw. ""EN 24" der Firma Chisso Corp. auf Basis der aus der DE-OS 29 37 700 bekannten Derivate von 2,3-Dicyanophenolen, d. h. Verbindungen der Formeln

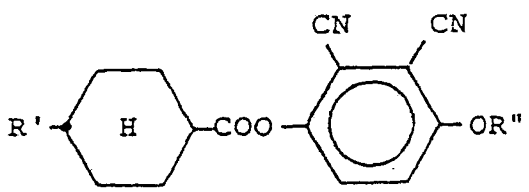

(I)

bzw.

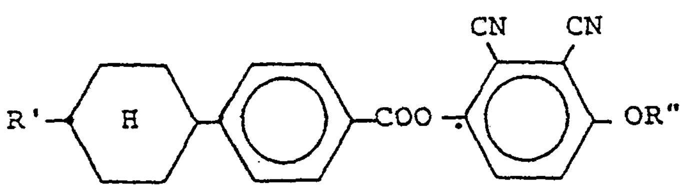

(II)

in welchen R' und R" Alkylgruppen sind. Es wurde gefunden, dass diese Verbindungen eine unzureichende fotochemische Stabilität aufweisen (siehe Tabelle I von Beispiel 5), was sich durch Zunahme der Leitfähigkeit der FK-Mischungen bei Einwirkung von Tageslicht-UV und damit durch eine nachteilige Erhöhung der Stromaufnahme von mit solchen FK-Mischungen arbeitenden Anzeigen äussert.

Aufgabe der Erfindung ist es, neue und verbesserte anisotrope Verbindungen mit negativer DKA, erhöhter fotochemischer Beständigkeit und höheren Klärpunkten anzugeben. Zur Aufgabe der Erfindung gehören auch FK-Mischungen mit insgesamt negativer DKA und verbesserter fotochemischer Beständigkeit, die sich für FK-Anzeigen eignen, welche wie die oben genannten GHD- und HND-Systeme nematische FK-Mischungen mit hoher negativer DKA benötigen.

Diese Aufgabe wird erfindungsgemäß gelöst durch neue anisotrope Verbindungen der Formel (I),

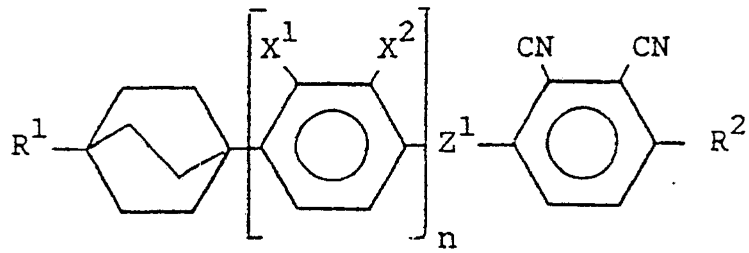

(1)

in der n Null oder Eins ist, $Z^1$ die Kovalenzbindung oder eine Brückengruppe der Formel $-CH_2O-$, $-CH_2CH_2-$ oder -COO- ist, $X^1$ und $X^2$ gleich oder verschieden und Wasserstoff- oder Halogenatome oder Nitrilgruppen darstellen, $R^1$ und $R^2$ Wasserstoffatome oder Alkyl-, Alkoxy-, Alkanoyloxy- oder Alkoxycarbonylgruppen mit jeweils 1 bis 12 C-Atomen im Alkylteil in gerader oder verzweigter und gegebenenfalls chiraler Kette bedeuten, wenn n gleich Null oder Eins ist, und wobei entweder $R^1$ oder $R^2$ einen Rest der Formel (1a), (1b) oder (1c)

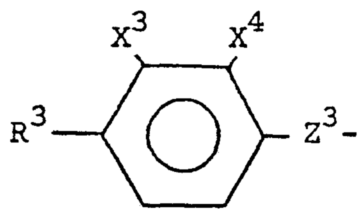

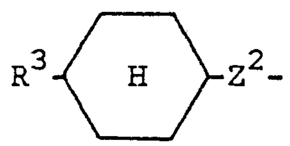

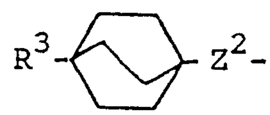

(1a) (1b) (1c)

bedeuten kann, wenn n Null ist, wobei $Z^2$ die für $Z^1$ angegebene Bedeutung hat, $Z^3$ die Kovalenzbindung oder eine Brückengruppe der Formel $-CH_2CH_2-$ oder -COO- ist, $R^3$ das Wasserstoffatom oder eine Alkyl-, Alkoxy-, Alkanoyloxygruppe oder Alkoxycarbonylgruppe mit 1 bis 12 C-Atomen im Alkylteil in gerader oder verzweigter und gegebenenfalls chiraler Kette ist, und $X^3$, $X^4$ eine der für $X^1$, $X^2$ angegebenen Bedeutungen haben, mit der Massgabe, dass keiner der im Molekül vorhandenen Benzolringe gleichzeitig direkt mit zwei Sauerstoffatomen und direkt mit Nitrilgruppen verbunden ist.

Der Einfachheit halber bedeuten im folgenden Bco eine Bicyclo[2.2.2]octan-1,4-diylgruppe, A eine 2,3-Dicyano-1,4-phenylengruppe, $A^1$ eine durch $X^1$ und $X^2$ in 2,3-Stellung disubstituierte 1,4-Phenylengruppe und Cyc eine trans-1,4-Cyclo-hexylengruppe. $R^1$, $R^2$, $R^3$, $R^4$, $Z^1$, $Z^2$, $Z^3$, $X^1$ und $X^2$ haben nachfolgend die angegebene Bedeutung.

Die Verbindungen der Formel (1) umfassen dementsprechend Verbindungen mit den Teilformeln (2) bis (9):

$R^3$-Bco-$Z^1$-A-$R^4$ (2)
$R^3$-Bco-$A^1$-$Z^1$-A-$R^4$ (3)
$R^3$-$A^1$-$Z^3$-Bco-$Z^1$-A-$R^4$ (4)
$R^3$-Cyc-$Z^2$-Bco-$Z^1$-A-$R^4$ (5)
$R^3$-Bco-$Z^2$-Bco-$Z^1$-A-$R^4$ (6)
$R^3$-Bco-$Z^1$-A-$Z^3$-$A^1$-$R^4$ (7)
$R^3$-Bco-$Z^1$-A-$Z^2$-Cyc-$R^4$ (8)
$R^3$-Bco-$Z^1$-A-$Z^2$-Bco-$R^4$ (9)

Gegenstand der Erfindung sind ferner Flüssigkristallmischungen mit einem Gehalt an mindestens einer Verbindung der Formeln (1) bis (9).

Alle erfindungsgemässen Verbindungen der Formeln (1) bis (9) enthalten wie die oben erwähnten bekannten Verbindungen der Formeln (I) bzw. (II) den 2,3-Dicyanophenylring und haben dementsprechend negative DKA-Werte; es wurde nun aber gefunden, dass die ungenügende fotochemische Beständigkeit der bekannten Verbindungen der Formeln (I) und (II) durch deren gemeinsames Strukturelement, nämlich den Kern der Formel (A)

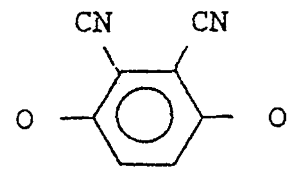

(A)

bedingt ist, was wahrscheinlich auf der Tendenz dieses Kerns zur Bildung chinoider bzw. chinoidähnlicher Bindungszust-nde

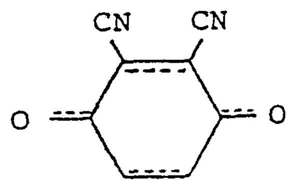

(B)

beruht.

Für die neuen Verbindungen der Formel (1) gilt erfindungsgemäss die Bestimmung, dass keiner der im Molekül vorhandenen nitrilsubstituierten Benzolringe direkt mit zwei Sauerstoffatomen verbunden ist, so dass chinoide Bindungs- oder Ladungsverteilungszustände am stets vorhandenen 2,3-Dicyano-phenyl-Anteil aber auch in allenfalls vorhandenen weiteren nitrilsubstituierten Benzolringen ausgeschaltet sind.

Erfindungsgemässe Verbindungen (1) können zwei- oder dreikernig sein. Die zweikernigen Verbindungen haben die in Formel (2) von Anspruch 2 definierte Form, d. h. die erfindungsgemässe Verbindung (2) besteht aus dem 2,3-Dicyanophenylkern und einem an diesem über das Brückenglied $Z^1$ (Kovalenzbindung oder eine der Gruppen $-CH_2O-$, $-CH_2CH_2-$, $-COO-$, gegebenenfalls in der dargestellten oder der entsprechend umgekehrten Form, d. h. $-OCH_2-$ bzw. $-OOC-$) gebundenen Bicyclo(2,2,2)octanring.

Bevorzugte zweikernige und bevorzugte dreikernige Strukturen enthalten die Flügelgruppe $R^3$ mit der in Anspruch 1 genannten Bedeutung, wobei $R^3 = H$ meist weniger bevorzugt ist. Die bevorzugten zwei- und dreikernigen erfindungsgemässen Verbindungen entsprechend den Formeln (2) bis (9) gemäss Definition in den Ansprüchen 2 bis 9 enthalten ferner eine weitere Flügelgruppe, welche eine der für $R^3$ genannten Bedeutungen hat.

Der Bicyclo(2,2,2)octanring

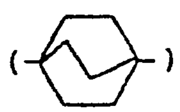

ist in den dreikernigen Verbindungen mindestens einmal (d. h. wenn n in Formel (1) gleich Eins ist) vorhanden, oder kann zweimal vorhanden sein, wenn n in Formel (1) Null ist und $R^1$ oder $R^2$ den cyclischen Rest der Formel (1c) bedeutet.

Die Verwendung von Bicyclo(2,2,2)octanringen in zwei- oder dreikernigen anisotropen Verbindungen mit positiver DKA ist z. B. aus GB-OS 2'027'027 bekannt. Hingegen sind bisher keine anisotropen Verbindungen mit negativer DKA bekannt geworden, die einen oder zwei Bicyclo(2,2,2)octanring(e) als Strukturelement enthalten und es war keineswegs naheliegend, dass die Kombination eines 2,3-Dicyanophenylringes mit mindestens einem Bicyclo(2,2,2)octanring für anisotrope Verbindungen mit negativer DKA besonders vorteilhaft sein würde. Dies ist aber der Fall; wie bei den zur Erfindung führenden Untersuchungen der Anmelderin festgestellt wurde, lässt sich die durch die zwei lateralen Nitrilgruppen des 2,3-Dicyanophenylringes allgemein bedingte Erniedrigung des Klärpunktes bei den bekannten Verbindungen mit negativer DKA bisher nur durch viskositätserhöhend wirkende Strukturelemente, z.B. Molekülverlängerung, kompensieren; überraschenderweise bieten die vorliegenden neuen Verbindungen (1) mit negativer DKA nicht nur eine im Vergleich zum Stand der Technik verbesserte fotochemische Stabilität, sondern auch eine vergleichsweise weniger ausgeprägte Klärpunktserniedrigung durch die lateralen Nitrilgruppen.

Wenn n in Formel (1) den Wert Null bedeutet, kann eine der Flügelgruppen $R^1$, $R^2$ ein cyclischer Rest der in Anspruch 1 angegebenen Formeln (1a), (1b) oder (1c) sein; die Definition Von $R^3$ entspricht den nicht-cyclischen Bedeutungen von $R^1$, $R^2$; die Brücke $Z^2$ hat eine der für $Z^1$ genannten Bedeutungen; $Z^3$ bedeutet ebenfalls eine Brücke, und zwar entweder die Kovalenzbindung oder eine der Gruppen $-CH_2CH_2-$ oder $-COO-$ (bzw. $-OOC-$).

Die Definition von $X^1$, $X^2$ in Formel (1) bzw. von $X^3$, $X^4$ in Formel (1a) umfasst neben Wasserstoff auch Halogenatome (F, Cl, Br, Jod) und die Nitrilgruppe. Erfindungsgemässe Verbindungen enthalten somit

mindestens die in Formel (1) dargestellten zwei lateralen Nitrilgruppen an dem stets vorhandenen Benzolring, können aber in dem gegebenenfalls vorhandenen zweiten Benzolring (d. h. wenn entweder n gleich Eins ist oder eine von $R^1$, $R^2$ den cyclischen Rest (1a) bedeutet) ein oder zwei zusätzliche laterale Substituenten der genannten Art (Halogen, Nitril) enthalten.

Wenn n in Formel (1) Eins darstellt, bedeuten beide $R^1$ und $R^2$ Wasserstoff oder Alkyl-, Alkoxy-, Alkänoyloxy- oder Alkoxycarbonylgruppen mit jeweils 1 bis 12 C-Atomen im Alkylteil in gerader oder verzweigter und gegebenenfalls chiraler Kette; wenn n in Formel (1) Null darstellt, kann - wie oben erläutert - entweder $R^1$ oder $R^2$ einen Rest der Formeln (1a), (1b) oder (1c) darstellen, worin $R^3$ die eben genannte nichtcyclische Bedeutung von $R^1$ bzw. $R^2$ hat. $R^4$ in den Formeln (2) bis (9) hat eine der für $R^3$ genannten Bedeutungen. Die Herstellung der erfindungsgemässen Verbindungen kann auf verschiedenen Synthesewegen erfolgen, wie sie an sich für die Herstellung von bekannten anisotropen Verbindungen bekannt sind, die mindestens einen Bicyclo(2,2,2)octanring und mindestens einen Benzolring enthalten. Beispiele wird hierbei eine entsprechende Bicyclo(2,2,2)octanverbindung mit einer 2,3-Dicyanophenylverbindung umgesetzt; alternativ können entsprechende Bicyclo(2,2,2)octan-Phenylverbindungen zur Einführung von Halogenatomen in 2,3-Stellung des Benzolringes halogeniert und die entsprechende Bicyclo(2,2,2)octan-2,3-dihalo-genphenylverbindung in die entsprechende 2,3-Dicyanoverbindung umgewandelt werden. Typische allgemeine Beispiele werden in den folgenden Schemata erläutert, in welchen "Ts" die Tosylgruppe ist und die übrigen Symbole die oben genannte Bedeutung haben:

(A) 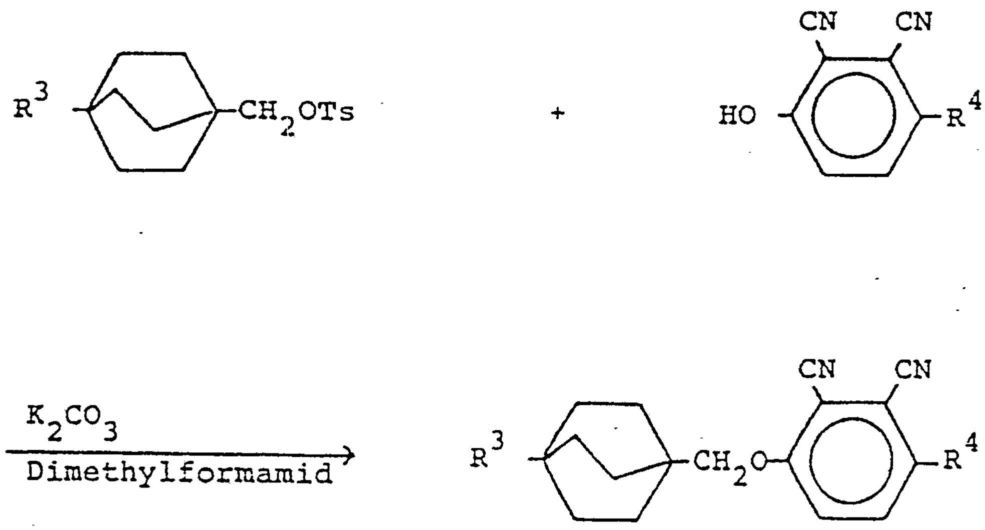


(B) 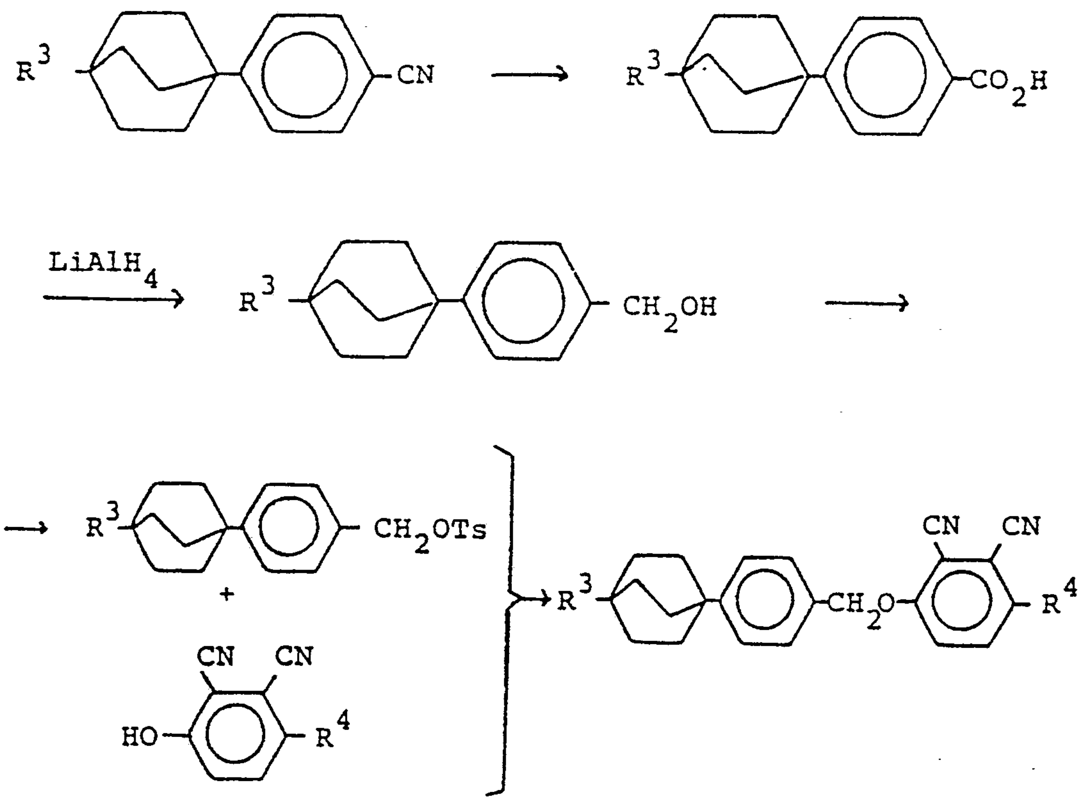

(C)

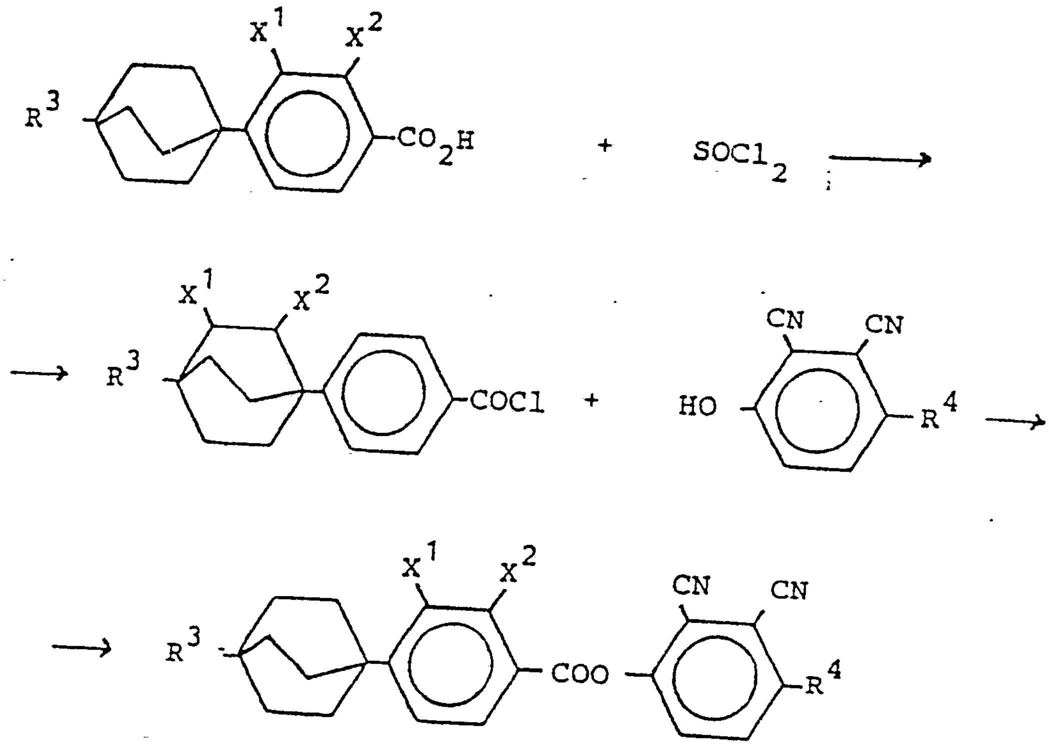


(D)

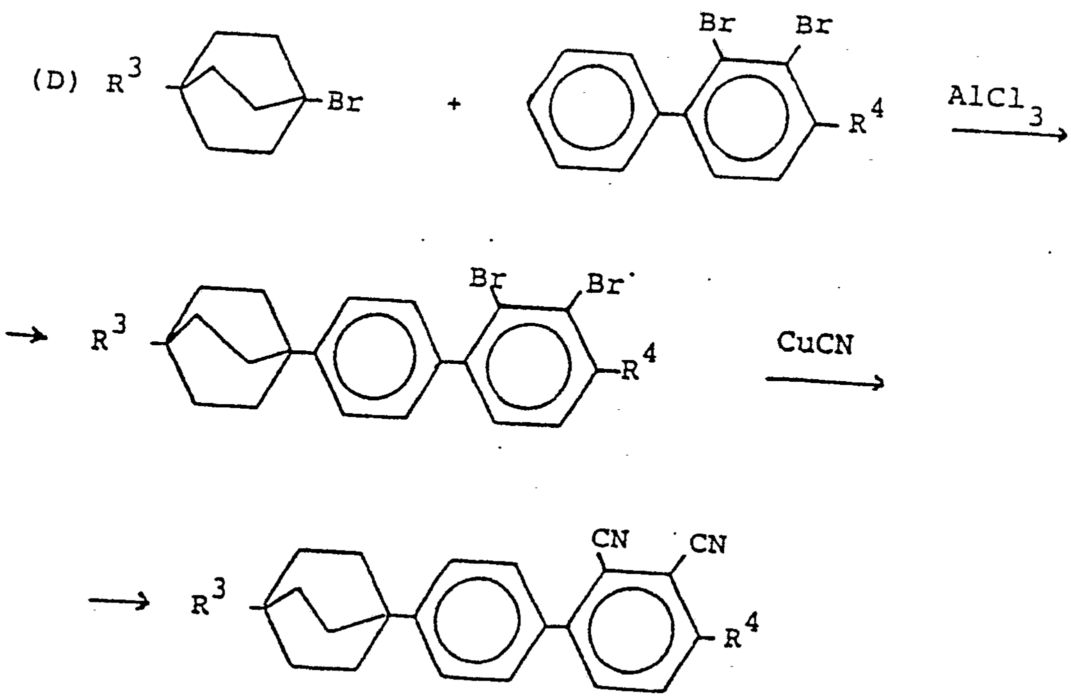

(E)

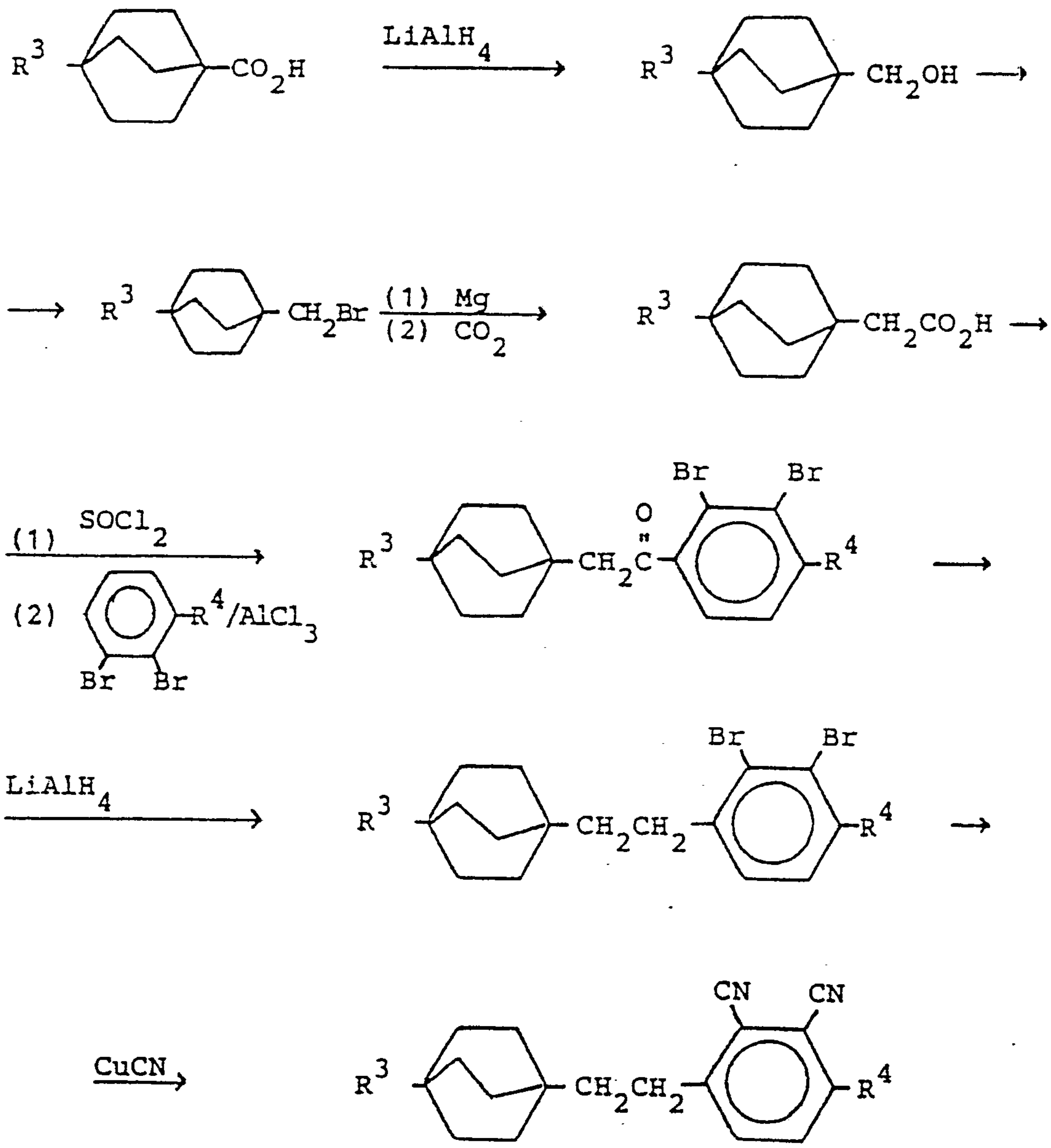

(F)

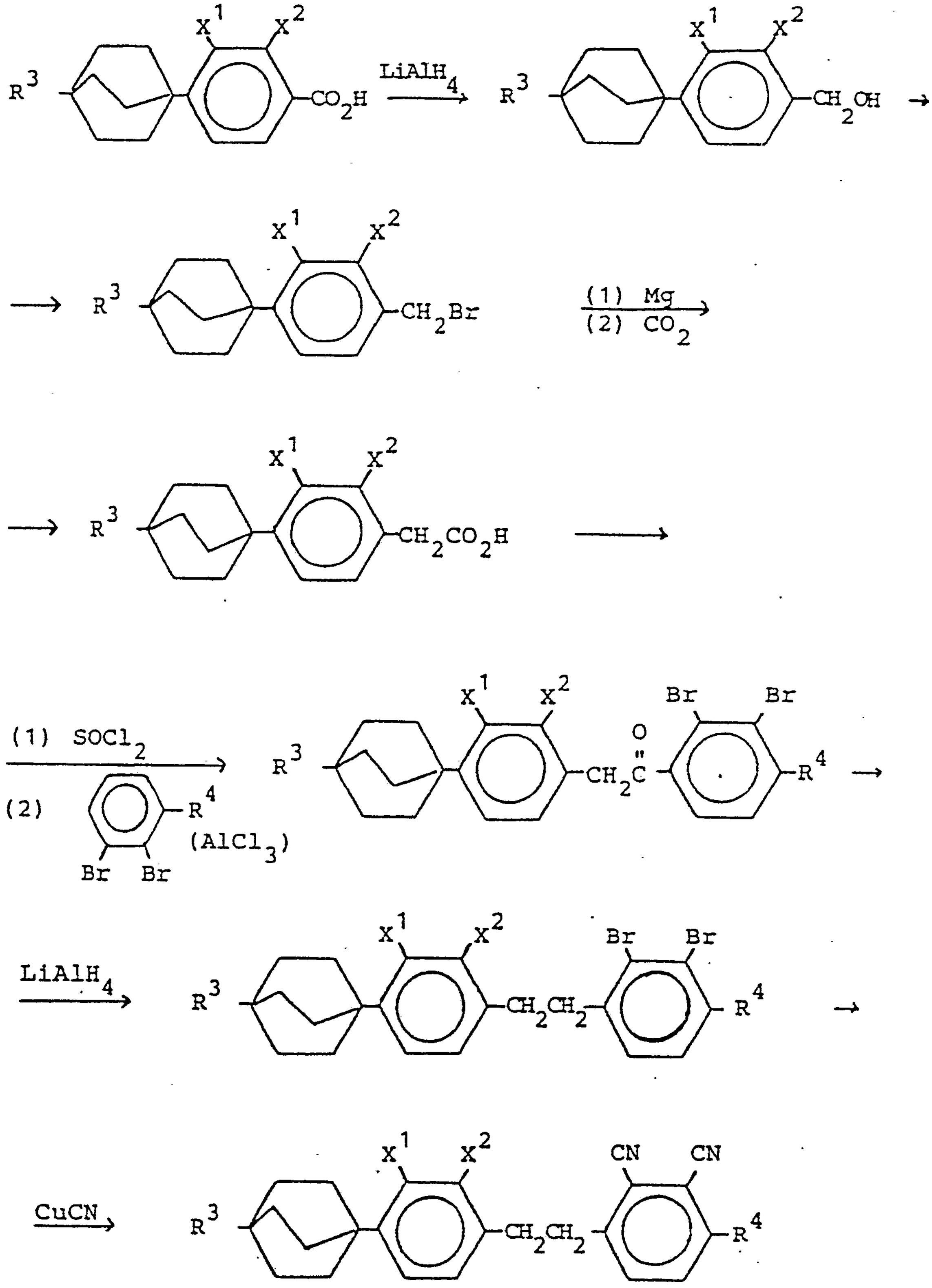

(G)

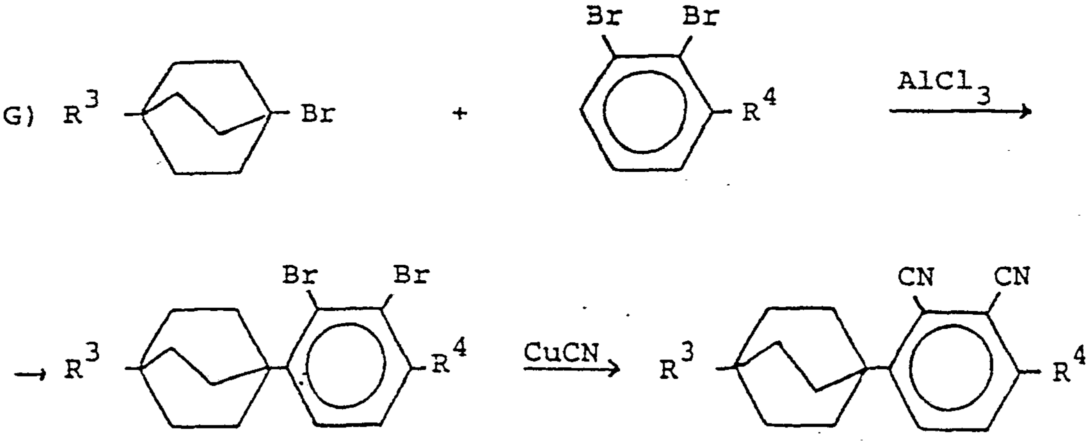


Geeignete Ausgangsverbindungen für die Umsetzungen gemäss den obigen Schemata sind aus der Literatur bekannt oder können analog wie die bekannten Verbindungen hergestellt werden. Ausgangsverbindungen mit Bicyclo(2,2,2)octangruppen bzw. deren Herstellung sind insbesondere in den veröffentlichten britischen Patentanmeldungen Nrn. 78/32350, 78/32351, 80/05351 und 81/02382 zu finden.

Die Erfindung wird anhand der folgenden Beispiele weiter erläutert.

**Beispiel 1**

**Herstellung von 2,3-Dicyano-4-pentylphenyl-4-pentylbicyclo(2,2,2)octan-1-carboxylat**

4-Pentylbicyclo(2,2,2)octan-1-carbonsäure (25 mM) wurde mit Thionylchlorid (40 ml) 30 min auf Rückflusstemperatur erwärmt. Das entstandene Säurechlorid wurde vom überschüssigen Thionylchlorid befreit. Das so gewonnene Säurechlorid wurde nun zu einer Lösung von 2,3-Dicyano-4-pentylphenol (Tm 142°C, 25 mM) in 100 ml Pyridin bei 80°C zugetropft. Nach Beendigung der Reaktion wurde das Gemisch in überschüssige verdünnte Salzsäure gegossen und mit Methylenchlorid extrahiert. Das aus dem Extrakt durch Eindampfen erhaltene Rohprodukt wurde umkristallisiert. Die so erhaltene Zielverbindung dieses Beispiels ist monotrop nematisch (K 84.0 N (36.1) I) und hat eine negative DKA.

**Beispiel 2**

**Herstellung von 2,3-Dicyano-4-pentylphenyl-4-(4-pentyl-1-bicyclo(2,2,2)octyl)-benzoat**

4-(4-Pentyl-1-bicyclo(2,2,2)octyl)-benzoesäure (36,5 mM) wurde mit Thionylchlorid (50 ml) 1 Std. unter Rückfluss gekocht. Das überschüssige Thionylchlorid wurde destilliert. Das entstandene Säurechlorid wurde zu einer Lösung von 36,5 mM 2,3-Dicyano-4-pentylphenol in 100 ml Pyridin zugetropft. Die Reaktionsmischung wurde bei 80°C bis zur Beendigung der Reaktion gerührt und dann auf verdünnte Salzsäure gegossen. Das Produkt wurde mit Methylenchlorid extrahiert. Zur Reinigung wurde das Produkt umkristallisiert. Es zeigt eine enantiotrope nematische Phase (K 130.9N 137.9 I) und hat eine negative DKA.

**Beispiel 3**

**Herstellung von 4-Propyl-1-bicyclo(2,2,2)octyl-methyl-2,3-dicyano-4-pentylphenyläther**

2,3-Dicyano-4-pentylphenol (0,04 M), Kaliumcarbonat (0,2 M) und 4-Propyl-1-bicyclo(2,2,2)octyl-methylbromid (0,04 M) wurden mit 50 ml Dimethylformamid versetzt. Das Gemisch wurde bei 8O° C 48 Std. gerührt. Dann wurd das Gemisch auf Raumtemperatur abgekühlt, in 200 ml Wasser gegossen und die organische Phase extrahiert. Der organische Extrakt wurde mit Wasser gewaschen und über Magnesiumsulfat getrocknet. Nach Eindampfen des Lösungsmittels wurde das Rohprodukt mehrmals umkristallisiert. Das reine Produkt hat einen hohen Schmelzpunkt von 133,1° C und eine negative DKA.

**Beispiel 4**

**Herstellung von 2,3-Dicyano-4-pentylphenyl-4-heptyl-bicyclo(2,2,2)octan-1-carboxylat**

Diese Verbindung wurde analog wie in Beispiel 1, jedoch unter Verwendung der entsprechenden 4-Heptyl-bicyclo(2,2,2)octan-1-carbonsäure hergestellt. Die erhaltene Verbindung ist monotrop nematisch (K 90 N (49) I) und hat eine negative DKA.

**Beispiel 5**

Zur Prüfung auf fotochemische Stabilität wurden folgende Verbindungen bzw. Mischungen unter gleichen Bedingungen mit einer Sonnenlichtlampe (1500 Watt) belichtet und auf Veränderung ihrer elektrischen Leitfähigkeit untersucht:

**Trägerverbindung**

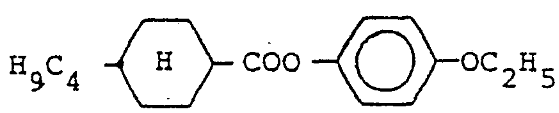

(A)

**Trägerverbindung**

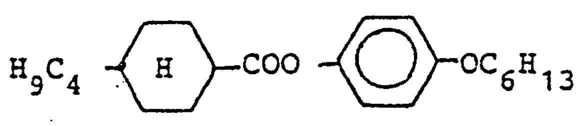

(B)

**Vergleichsverbindung mit negativer DKA**

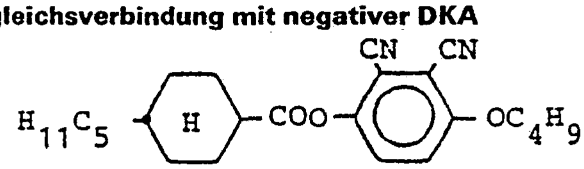

(53)

**Erfindungsgemässe Verbindung mit negativer DKA**

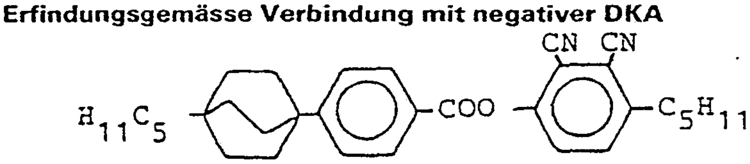

(30)

sowie eine kommerzielle FK-Mischung der Firma Chisso Corp., Typ EN 24, mit negativer DKA. Die Ergebnisse sind in der folgenden Tabelle I zusammengestellt.

TABELLE I

| Bestrahlungsdauer mit Sonnenlichtlampe von 1500 Watt (Std.) | Leitfähigkeitswerte (µS) gemessen an: Träger (A) | Träger (B) | Mischung aus 10 Mol% erfindungsg. Verb. (30) und 90 Mol% (A) | Mischung aus 11 Mol% Vergleichsverb. (53) und 89 Mol% (B) | kommerzielle Mischung "EN 24" |
|---|---|---|---|---|---|
| 0 | 0,19 | 0,03 | 0,82 | 0,07 | 0,2 |
| 10 | 0,20 | 0,03 | 0,83 | 0,09 | 0,4 |
| 20 | 0,20 | 0,03 | 0,84 | 0,12 | 0,7 |
| 40 | 0,20 | 0,03 | 0,85 | 0,17 | 1,1 |
| 60 | 0,20 | 0,03 | 0,87 | 0,22 | 1,6 |
| 80 | 0,20 | 0,03 | 0,88 | 0,27 | 2,0 |
| 100 | 0,20 | 0,03 | 0,90 | 0,32 | 2,5 |

Die Werte von Tabelle I zeigen zunächst, dass die als Trägerverbindungen bei den Messungen verwendeten anisotropen Verbindungen (A) und (B) ohne laterale Nitrilgruppen auch dann fotochemisch stabil sind, wenn der Phenylrest direkt mit zwei Sauerstoffverbindungen verknüpft ist; obwohl chinoide Bindungszustände bei (A) und (B) strukturell nicht ausgeschlossen sind, führt dies ohne die lateralen Nitrilgruppen offensichtlich nicht zu fotochemischer Instabilität.

Die nicht-erfindungsgemässe Verbindung (53) mit einem 2,3-Dicyanophenylkern, der direkt mit zwei Sauerstoffatomen verbunden ist, erweist sich ebenso wie die kommerzielle Mischung EN 24 als fotochemisch instabil, indem der Leitfähigkeitswert nach 100 Stunden Bestrahlung auf das 4,5-Fache bzw. 12-Fache des Ausgangswertes gestiegen war, während die Mischung mit der erfindungsgemässen Verbindung mit negativer DKA nach 100 Stunden Bestrahlung eine nur geringfügig, d. h. nur um etwa 10 % höhere Leitfähigkeit zeigte.

Die neuen erfindungsgemässen Verbindungen (1) mit negativer DKA können grundsätzlich gleich wie die z. B. aus DE-OS 29 37 700 bekannten anisotropen 2,3-Dicyanoverbindungen mit negativer DKA für FK-Mischungen mit insgesamt negativer DKA, aber mit dem Vorteil einer wesentlich höheren fotochemischen Beständigkeit, verwendet werden, und zwar zusammen mit beispielsweise zum Betrieb von GHD oder HND bekannten Zusätzen an weiteren anisotropen Verbindungen, Farbstoffen, optisch aktiven Zusätzen und dergleichen.

Im allgemeinen können die erfindungsgemässen anisotropen Verbindungen einen wesentlichen oder sogar überwiegenden Teil von FK-Mischungen mit insgesamt negativer DKA ausmachen, z.B. bis etwa 60 Mol% Verbindungen der Formel (1) als Anteil der Mischung. Vorzugsweise werden dabei meist mehrere verschiedene Verbindungen (1), z. B. in Anteilen von jeweils 1 bis 30 Mol%, bezogen auf die Mischung, verwendet.

Die folgenden weiteren Verbindungen gemäss Formel (1) sind zu nennen:

2,3-Dicyano-4-propylphenyl-4-propylbicyclo(2,2,2)octan-1-carboxylat
2,3-Dicyano-4-pentylphenyl-4-propylbicyclo(2,2,2)octan-1-carboxylat
2,3-Dicyano-4-pentylphenyl-4-pentylbicyclo(2,2,2)octan-1-carboxylat
2,3-Dicyano-4-propylphenyl-4-heptylbicyclo(2,2,2)octan-1-carboxylat
2,3-Dicyano-4-pentylphenyl-4-heptylbicyclo(2,2,2)octan-1-carboxylat
2,3-Dicyano-4-propylphenyl-4-(4-propyl-1-bicyclo(2,2,2)octyl)-benzoat
2,3-Dicyano-4-pentylphenyl-4-(4-propyl-1-bicyclo(2,2,2)octyl)-benzoat
2,3-Dicyano-4-propylphenyl-4-(4-pentyl-1-bicyclo(2,2,2)octyl)-benzoat
2,3-Dicyano-4-pentylphenyl-4-(4-pentyl-1-bicyclo(2,2,2)octyl)-benzoat
1-(4-Propyl-1-bicyclo(2,2,2)octyl)-2-(2,3-dicyano-4-propylphenyl)-ethan
1-(4-Propyl-1-bicyclo(2,2,2)octyl)-2-(2,3-dicyano-4-pentylphenyl)-ethan
1-(4-Pentyl-1-bicyclo(2,2,2)octyl)-2-(2,3-dicyano-4-pentylphenyl)-ethan
1-(4-Propyl-1-bicyclo(2,2,2)octyl)-2-(2,3-dicyano-4-ethoxyphenyl)-ethan
1-(4-Propyl-1-bicyclo(2,2,2)octyl)-2-(2,3-dicyano-4-butoxyphenyl)-ethan
1-(4-Pentyl-1-bicyclo(2,2,2)octyl)-2-(2,3-dicyano-4-ethoxyphenyl)-ethan
1-(4-Pentyl-1-bicyclo(2,2,2)octyl)-2-(2,3-dicyano-4-butoxyphenyl)-ethan
1-(4-Propyl-1-bicyclo(2,2,2)octyl)-2-(2,3-dicyano-4-propanoyloxyphenyl)-ethan
1-(4-Propyl-1-bicyclo(2,2,2)octyl)-2-(2,3-dicyano-4-pentanoyloxyphenyl)-ethan
1-(4-Pentyl-1-bicyclo(2,2,2)octyl)-2-(2,3-dicyano-4-propanoyloxyphenyl)-ethan
1-(4-Pentyl-1-bicyclo(2,2,2)octyl)-2-(2,3-dicyano-4-pentanoyloxyphenyl)-ethan
1-(4-(4-Propyl-1-bicyclo(2,2,2)octyl)phenyl)-2-(2,3-dicyano-4-propylphenyl)-ethan
1-(4-(4-Propyl-1-bicyclo(2,2,2)octyl)phenyl)-2-(2,3-dicyano-4-pentylphenyl)-ethan
1-(4-(4-Pentyl-1-bicyclo(2,2,2)octyl)phenyl)-2-(2,3-dicyano-4-propylphenyl)-ethan
1-(4-(4-Pentyl-1-bicyclo(2,2,2)octyl)phenyl)-2-(2,3-dicyano-4-pentylphenyl)-ethan
1-(4-(4-Propyl-1-bicyclo(2,2,2)octyl)phenyl)-2-(2,3-dicyano-4-ethoxyphenyl)-ethan
1-(4-(4-Propyl-1-bicyclo(2,2,2)octyl)phenyl)-2-(2,3-dicyano-4-butoxyphenyl)-ethan
1-(4-(4-Pentyl-1-bicyclo(2,2,2)octyl)phenyl)-2-(2,3-dicyano-4-ethoxyphenyl)-ethan
1-(4-(4-Pentyl-1-bicyclo(2,2,2)octyl)phenyl)-2-(2,3-dicyano-4-butoxyphenyl)-ethan
1-(4-(Propyl-1-bicyclo(2,2,2)octyl)phenyl)-2-(2,3-dicyano-4-propanoyloxyphenyl)-ethan
1-(4-(Propyl-1-bicyclo(2,2,2)octyl)phenyl)-2-(2,3-dicyano-4-pentanoyloxyphenyl)-ethan
1-(4-(Pentyl-1-bicyclo(2,2,2)octyl)phenyl)-2-(2,3-dicyano-4-propanoyloxyphenyl)-ethan
1-(4-(Pentyl-1-bicyclo(2,2,2)octyl)phenyl)-2-(2,3-dicyano-4-pentanoyloxyphenyl)-ethan
4-Propyl-1-bicyclo(2,2,2)octylmethyl-2,3-dicyano-4-propylphenyl-äther
4-Propyl-1-bicyclo(2,2,2)octylmethyl-2,3-dicyano-4-pentylphenyl-äther
4-Pentyl-1-bicyclo(2,2,2)octylmethyl-2,3-dicyano-4-propylphenyl-äther
4-Pentyl-1-bicyclo(2,2,2)octylmethyl-2,3-dicyano-4-pentylphenyl-äther
1-Propyl-4-(2,3-dicyano-4-propylphenyl)-bicyclo(2,2,2)octan
1-Propyl-4-(2,3-dicyano-4-pentylphenyl)-bicyclo(2,2,2)octan
1-Pentyl-4-(2,3-dicyano-4-propylphenyl)-bicyclo(2,2,2)octan
1-Pentyl-4-(2,3-dicyano-4-pentylphenyl)-bicyclo(2,2,2)octan
1-Propyl-4-(2,3-dicyano-4-ethoxyphenyl)-bicyclo(2,2,2)octan
1-Propyl-4-(2,3-dicyano-4-butoxyphenyl)-bicyclo(2,2,2)octan
1-Pentyl-4-(2,3-dicyano-4-ethoxyphenyl)-bicyclo(2,2,2)octan
1-Pentyl-4-(2,3-dicyano-4-butoxyphenyl)-bicyclo(2,2,2)octan
1-Propyl-4-(2,3-dicyano-4-propanoyloxyphenyl)-bicyclo(2,2,2)-octan

1-Propyl-4-(2,3-dicyano-4-pentanoyloxyphenyl)-bicyclo(2,2,2)-octan
1-Pentyl-4-(2,3-dicyano-4-propanoyloxyphenyl)-bicyclo(2,2,2)-octan
1-Pentyl-4-(2,3-dicyano-4-pentanoyloxyphenyl)-bicyclo(2,2,2)-octan
1-(4-Propyl-1-bicyclo(2,2,2)octyl)-4-(2,3-dicyano-4-propylphenyl)-benzol
1-(4-Propyl-1-bicyclo(2,2,2)octyl)-4-(2,3-dicyano-4-pentylphenyl)-benzol
1-(4-Pentyl-1-bicyclo(2,2,2)octyl)-4-(2,3-dicyano-4-propylphenyl)-benzol
1-(4-Pentyl-1-bicyclo(2,2,2loctyl)-4-(2,3-dicyano-4-pentylphenyl)-benzol
1-(4-Propyl-1-bicyclo(2,2,2)octyl)-4-(2,3-dicyano-4-butoxyphenyl)-benzol
1-(4-Pentyl-1-bicyclo(2,2,2)octyl)-4-(2,3-dicyano-4-butoxyphenyl)-benzol
1-(4-Pentyl-1-bicyclo(2,2,2)octyl)-4-(2,3-dicyano-4-propanoyloxyphenyl)-benzol
1-(4-Pentyl-1-bicyclo(2,2,2)octyl)-4-(2,3-dicyano-4-butanoyloxyphenyl)-benzol
2,3-Dicyano-4-propylphenyl-4-(4-propyl-1-bicyclo(2,2,2)octyl)-2-fluorobenzoat
2,3-Dicyano-4-propylphenyl-4-(4-propyl-1-bicyclo(2,2,2)octyl)-2-chlorobenzoat
2,3-Dicyano-4-propylphenyl-4-(4-propyl-1-bicyclo(2,2,2)octyl)-2-bromobenzoat
2,3-Dicyano-4-pentylphenyl-4-(4-pentyl-1-bicyclo(,2,2)octyl)-3-fluorobenzoat
2,3-Dicyano-4-pentylphenyl-4-(4-pentyl-1-bicyclo(2,2,2)octyl)-3-chlorobenzoat
2,3-Dicyano-4-pentylphenyl-4-(4-pentyl-1-bicyclo(2,2,2)octyl)-3-bromobenzoat
2,3-Dicyano-4-pentylphenyl-4-(4-pentyl-1-bicyclo(2,2,2)octyl)-3-cyanobenzoat
1-(4-(4-Propyl-1-bicyclo(2,2,2)octyl)-2-fluorophenyl)-2-(2,3-dicyano-4-propylphenyl)-ethan
1-(4-(4-Propyl-1-bicyclo(2,2,2)octyl)-2-chlorophenyl)-2-(2,3-dicyano-4-propylphenyl)-ethan
1-(4-(4-Propyl-1-bicyclo(2,2,2)octyl)-2-bromophenyl)-2-(2,3-dicyano-4-propylphenyl)-ethan
1-(4-(4-Propyl-1-bicyclo(2,2,2)octyl)-2-fluorophenyl)-2-(2,3-dicyano-4-butoxyphenyl)-ethan
1-(4-(4-Propyl-1-bicyclo(2,2,2)octyl)-2-chlorophenyl)-2-(2,3-dicyano-4-butoxyphenyl)-ethan
1-(4-(4-Propyl-1-bicyclo(2,2,2)octyl)-2-bromophenyl)-2-(2,3-dicyano-4-butoxyphenyl)-ethan
1-(4-(4-Propyl-1-bicyclo(2,2,2)octyl)-2-fluorophenyl)-2-(2,3-dicyano-4-propanoyloxyphenyl)-ethan
1-(4-(4-Propyl-1-bicyclo(2,2,2)octyl)-2-chlorophenyl)-2-(2,3-dicyano-4-propanoyloxyphenyl)-ethan
1-(4-(4-Propyl-1-bicyclo(2,2,2)octyl)-2-bromophenyl)-2-(2,3-dicyano-4-propanoyloxyphenyl)-ethan
1-(4-(4-Pentyl-1-bicyclo(2,2,2)octyl)-3-fluorophenyl)-2-(2,3-dicyano-4-pentylphenyl)-ethan
1-(4-(4-Pentyl-1-bicyclo(2,2,2)octyl)-3-chlorophenyl)-2-(2,3-dicyano-4-pentylphenyl)-ethan
1-(4-(4-Pentyl-1-bicyclo(2,2,2)octyl)-3-bromophenyl)-2-(2,3-dicyano-4-pentylphenyl)-ethan
1-(4-(4-Pentyl-1-bicyclo(2,2,2)octyl)-2,3-difluorophenyl)-2-(2,3-dicyano-4-pentylphenyl)-ethan
1-(4-(4-Pentyl-1-bicyclo(2,2,2)octyl)-3-fluorophenyl)-2-(2,3-dicyano-4-butoxyphenyl)-ethan
1-(4-(4-Pentyl-1-bicyclo(2,2,2)octyl)-3-chlorophenyl)-2-(2,3-dicyano-4-butoxyphenyl)-ethan
1-(4-(4-Pentyl-1-bicyclo(2,2,2)octyl)-3-bromophenyl)-2-(2,3-dicyano-4-butoxyphenyl)-ethan
1-(4-(4-Pentyl-1-bicyclo(2,2,2)octyl)-2,3-dichlorophenyl-2-(2,3-dicyano-4-butoxyphenyl)-ethan
1-(4-(4-Pentyl-1-bicyclo(2,2,2)octyl)-3-fluorophenyl)-2-(2,3-dicyano-4-propanoyloxyphenyl)-ethan
1-(4-(4-Pentyl-1-bicyclo(2,2,2)octyl)-3-chlorophenyl)-2-(2,3-dicyano-4-propanoyloxyphenyl)-ethan
1-(4-(4-Pentyl-1-bicyclo(2,2,2)octyl)-3-bromophenyl)-2-(2,3-dicyano-4-propanoyloxyphenyl)-ethan.
1-(4-(4-Pentyl-1-bicyclo(2,2,2)octyl)-3-cyanophenyl)-2-(2,3-dicyano-4-propanoyloxyphenyl)-ethan
1-(4-Propyl-1-bicyclo(2,2,2)octyl)-4-(2,3-dicyano-4-propyl-phenyl)-3-fluorobenzol
1-(4-Propyl-1-bicyclo(2,2,2)octyl)-4-(2,3-dicyano-4-propyl-phenyl)-3-chlorobenzol
1-(4-Propyl-1-bicyclo(2,2,2)octyl)-4-(2,3-dicyano-4-propyl-phenyl)-3-bromobenzol
1-(4-Propyl-1-bicyclo(2,2,2)octyl)-4-(2,3-dicyano-4-propyl-phenyl)-2,3-dicyanobenzol
1-(4-Propyl-1-bicyclo(2,2,2)octyl)-4-(2,3-dicyano-4-butoxy-phenyl)-3-fluorobenzol
1-(4-Propyl-1-bicyclo(2,2,2)octyl)-4-(2,3-dicyano-4-butoxy-phenyl)-3-chlorobenzol
1-(4-Propyl-1-bicyclo(2,2,2)octyl)-4-(2,3-dicyano-4-butoxy-phenyl)-3-bromobenzol
1-(4-Propyl-1-bicyclo(2,2,2)octyl)-4-(2,3-dicyano-4-butoxy-phenyl)-2,3-dicyanobenzol
1-(4-Propyl-1-bicyclo(2,2,2)octyl)-4-(2,3-dicyano-4-propa-noyloxyphenyl)-3-fluorobenzol
1-(4-Propyl-1-bicyclo(2,2,2)octyl)-4-(2,3-dicyano-4-propa-noyloxyphenyl)-3-chlorobenzol
1-(4-Propyl-1bicylo(2,2,2)octyl)-4-(2,3-dicyano-4-propa noyloxyphenyl)-3-bromobenzol
1-(4-Propyl-1-bicyclo(2,2,2)octyl)-4-(2,3-dicyano-4-propa-noyloxyphenyl)-2,3-dicyanobenzol
1-(4-Pentyl-1-bicyclo(2,2,2)octyl)-4-(2,3-dicyano-4-pentyl-phenyl)-2-fluorobenzol
1-(4-Pentyl-1-bicyclo(2,2,2)octyl)-4-(2,3-dicyano-4-pentyl-phenyl)-2-chlorobenzol
1-(4-Pentyl-1-bicyclo(2,2,2)octyl)-4-(2,3-dicyano-4-pentyl-phenyl)-2-bromobenzol
1-(4-Pentyl-1-bicyclo(2,2,2)octyl)-4-(2,3-dicyano-4-pentyl-phenyl)-2,3-difluorobenzol
1-(4-Pentyl-1-bicyclo(2,2,2)octyl)-4-(2,3-dicyano-4-butoxy-phenyl)-2-fluorobenzol
1-(4-Pentyl-1-bicyclo(2,2,2)octyl)-4-(2,3-dicyano-4-butoxy-phenyl)-2-chlorobenzol
1-(4-Pentyl-1-bicyclo(2,2,2)octyl)-4-(2,3-dicyano-4-butoxy phenyl)-2-bromobenzol
1-(4-Pentyl-1-bicyclo(2,2,2)octyl)-4-(2,3-dicyano-4-butoxy-phenyl)-2,3-dichlorobenzol
1-(4-Pentyl-1-bicyclo(2,2,2)octyl)-4-(2,3-dicyano-4-propa-noyloxyphenyl)-2-fluorobenzol
1-(4-Pentyl-1-bicyclo(2,2,2)octyl)-4-(2,3-dicyano-4-propa-noyloxyphenyl)-2-chlorobenzol
1-(4-Pentyl-1-bicyclo(2,2,2)octyl)-4-(2,3-dicyano-4-propa-noyloxyphenyl)-2-bromobenzol
1-(4-Pentyl-1-bicyclo(2,2,2)octyl)-4-(2,3-dicyano-4-propa-noyloxyphenyl)-2,3-dibromobenzol
1-(2,3-Dicyano-4-propylphenyl)-4-(4-propyl-1-bicyclo(2,2,2)-octyl)-2,3-dicyanobenzoat
1-(2,3-Dicyano-4-pentylphenyl)-4-(4-propyl-1-bicyclo(2,2,2)-octyl)-2,3-dicyanobenzoat

1-(2,3-Dicyano-4-propylphenyl)-4-(4-pentyl-1-bicyclo(2,2,2)-octyl)-2,3-dicyanobenzoat
1-(2,3-Dicyano-4-pentylphenyl)-4-(4-pentyl-1-bicyclo(2,2,2)-octyl)-2,3-dicyanobenzoat
1-[4-(4-Propyl-1-bicyclo(2,2,2)octyl)-2,3-dicyanophenyl]-2-(2,3-dicyano-4-propylphenyl)-ethan
1-[4-(4-Propyl-1-bicyclo(2,2,2)octyl)-2,3-dicyanophenyl]-2-(2,3-dicyano-4-pentylphenyl)-ethan
1-[4-(4-Propyl-1-bicyclo(2,2,2)octyl)-2,3-dicyanophenyl]-2-(4-butoxy-2,3-dicyanophenyl)-ethan;
1-[4-(4-Propyl-1-bicyclo(2,2,2)octyl)-2,3-dicyanophenyl]-2-(4-propanoyloxy-2,3-dicyanophenyl)-ethan
1-[4-(4-Pentyl-1-bicyclo(2,2,2)octyl)-2,3-dicyanophenyl]-2-(2,3-dicyano-4-propylphenyl)-ethan
1-[4-(4-Pentyl-1-bicyclo(2,2,2)octyl)-2,3-dicyanophenyl]-2-(2,3-dicyano-4-pentylphenyl)-ethan
1-[4-(4-Pentyl-1-bicyclo(2,2,2)octyl)-2,3-dicyanophenyl]-2-(4-butoxy-2,3-dicyanophenyl)-ethan
1-[4-(4-Pentyl-1-bicyclo(2,2,2)octyl)-2,3-dicyanophenyl]-2-(4-propanoyloxy-2,3-dicyanophenyl)-ethan
4-(4-Propyl-1-bicyclo(2,2,2)octyl)-2,3-dicyano-4'-propyl-biphenyl
4-(4-Pentyl-1-bicyclo(2,2,2)octyl)-2,3-dicyano-4'-propyl-biphenyl
4-(4-Propyl-1-bicyclo(2,2,2)octyl-2,3-dicyano-4'-pentyl-biphenyl
4-(4-Pentyl-1-bicyclo(2,2,2)octyl-2,3-dicyano-4'-pentyl-biphenyl
4-(4-Propyl-1-bicyclo(2,2,2)octyl)-2,3-dicyano-4'-butoxy-biphenyl
4-(4-Pentyl-1-bicyclo(2,2,2)octyl)-2,3-dicyano-4'-butoxy-biphenyl
4'-[4-(4-Propyl-1-bicyclo(2,2,2)octyl)-2,3-dicyano-biphenyl]-butanoat
4'-[4-(4-Pentyl-1-bicyclo(2,2,2)octyl)-2,3-dicyano-biphenyl]-butanoat
4-(4-Propyl-1-bicyclo(2,2,2)octyl)-2,3-dicyano-3'-fluoro-4'-propyl-biphenyl
4-(4-Pentyl-1-bicyclo(2,2,2)octyl)-2,3-dicyano-3'-fluoro-4'-propyl-biphenyl
4-(4-Propyl-1-bicyclo(2,2,2)octyl)-2,3-dicyano-3'-fluoro-4'-pentyl-biphenyl
4-(4-Pentyl-1-bicyclo(2,2,2)octyl)-2,3-dicyano-3'-fluoro-4'-pentyl-biphenyl
4-(4-Propyl-1-bicyclo(2,2,2)octyl)-2,3-dicyano-4'-butoxy-3'-fluoro-biphenyl
4-(4-Pentyl-1-bicyclo(2,2,2)octyl)-2,3-dicyano-4'butoxy-3'-fluoro-biphenyl
4'-[4-(4-Propyl-1-bicyclo(2,2,2)octyl)-2,3-dicyano-3'-fluoro-biphenyl]-butanoat
4'-[4-(4-Pentyl-1-bicyclo(2,2,2)octyl)-2,3-dicyano-3'-fluoro-biphenyl]-butanoat
4-(4-propyl-1-bicyclo(2,2,2)octyl)-2,3-dicyano-3'-chloro-4'-propyl-biphenyl
4-(4-Pentyl-1-bicyclo(2,2,2)octyl)-2,3-dicyano-3'-chloro-4'-propyl-biphenyl
4-(4-Propyl-1-bicyclo(2,2,2)octyl)-2,3-dicyano-3'-chloro-4'-pentyl-biphenyl
4-(4-Pentyl-1-bicyclo(2,2,2)octyl)-2,3-dicyano-4'-chloro-4'-pentyl-biphenyl
4-(4-Propyl-1-bicyclo(2,2,2)octyl)-2,3-dicyano-4'-butoxy-3'-chloro-biphenyl
4-(4-Pentyl-1-bicyclo(2,2,2)octyl)-2,3-dicyano-4'-butoxy-3'-chloro-biphenyl
4'-[4-(4-Propyl-1-bicyclo(2,2,2)octyl)-2,3-dicyano-3'-chloro-biphenyl]-butanoat
4'-[4-(4-Pentyl-1-bicyclo(2,2,2)octyl)-2,3-dicyano-3'-chloro-biphenyl]-butanoat
4-(4-Propyl-1-bicyclo(2,2,2)octyl)-2,3-dicyano-3'-bromo-4'-propyl-biphenyl
4-(4-Propyl-1-bicyclo(2,2,2)octyl)-2,3-dicyano-3'-bromo-4'-pentyl-biphenyl
4-(4-Propyl-1-bicyclo(2,2,2)octyl)-2,3-dicyano-3'-bromo-4'-butoxy-biphenyl
4-(4-Pentyl-1-bicyclo(2,2,2)octyl)-2,3-dicyano-3'-bromo-4'-propyl-biphenyl
4-(4-Pentyl-1-bicyclo(2,2,2)octyl)-2,3-dicyano-3'-bromo-4'-pentyl-biphenyl
4-(4-Pentyl-1-bicyclo(2,2,2)octyl)-2,3-dicyano-3'-bromo-4'-butoxy-biphenyl
4'-[4-(4-Propyl-1-bicyclo(2,2,2)octyl)-2,3-dicyano-3'-bromo-biphenylyl]-butanoat
4'-[4-(4-Pentyl-1-bicyclo(2,2,2)octyl)-2,3-dicyano-3'-bromo-biphenylyl]-butanoat
4-(4-Propyl-1-bicyclo(2,2,2)octyl)-2,3-dicyano-4'-butoxy-3'-cyano-biphenyl
4-(4-Propyl-1-bicyclo(2,2,2)octyl)-2,3-dicyano-3'-cyano-4'-propyl-biphenyl
4-(4-Propyl-1-bicyclo(2,2,2)octyl)-2,3-dicyano-3'-cyano-4'-pentyl-biphenyl
4-(4-Pentyl-1-bicyclo(2,2,2)octyl)-2,3-dicyano-4'-butoxy-3'-cyano-biphenyl
4-(4-Pentyl-1-bicyclo(2,2,2)octyl)-2,3-dicyano-3'-cyano-4'-propyl-biphenyl
4-(4-Pentyl-1-bicyclo(2,2,2)octyl)-2,3-dicyano-3'-cyano-4'-pentyl-biphenyl
4'-[4-(4-Propyl-1-bicyclo(2,2,2)octyl)-2,3-dicyano-3'-cyano-biphenylyl]-butanoat
4'-[4-(4-Pentyl-1-bicyclo(2,2,2)octyl)-2,3-dicyano-3'-cyano-biphenylyl]-butanoat
1-[4-(4-Propyl-1-bicyclo(2,2,2)-octyl)-2,3-dicyanophenyl]-2-(4-propylphenyl)-ethan
1-[4-(4-Propyl-1-bicyclo(2,2,2)octyl)-2,3-dicyanophenyl]-2-(4-pentylphenyl)-ethan
1-[4-(4-Propyl-1-bicyclo(2,2,2)octyl)-2,3-dicyanophenyl]-2-(4-butoxyphenyl)-ethan
1-[4-(4-Propyl-1-bicyclo(2,2,2)octyl)-2,3-dicyanophenyl]-2-(4-propylcarbonyloxyphenyl)-ethan
1-[4-(4-Pentyl-1-bicyclo(2,2,2)octyl)-2,3-dicyanophenyl]-2-(4-propylphenyl)-ethan
1-[4-(4-Pentyl-1-bicyclo(2,2,2)octyl)-2,3-dicyanophenyl]-2-(4-pentylphenyl)-ethan
1-[4-(4-Pentyl-1-bicyclo(2,2,2)octyl)-2,3-dicyanophenyl]-2-(4-butoxyphenyl)-ethan
1-[4-(4-Pentyl-1-bicyclo(2,2,2)octyl)-2,3-dicyanophenyl]-2-(4-propanoyloxyphenyl)-ethan
1-[4-(4-Propyl-1-bicyclo(2,2,2)octyl)-2,3-dicyanophenyl]-2-(3-fluoro-4-propylphenyl)-ethan
1-[4-(4-Propyl-1-bicyclo(2,2,2)octyl)-2,3-dicyanophenyl]-2-(3-fluoro-4-pentylphenyl)-ethan
1-[4-(4-Propyl-1-bicyclo(2,2,2)octyl)-2,3-dicyanophenyl]-2-(4-butoxy-3-fluorophenyl)-ethan
1-[4-(4-Propyl-1-bicyclo(2,2,2)octyl)-2,3-dicyanophenyl]-2-(3-fluoro-4-propanoyloxyphenyl)-ethan
1-[4-(4-Pentyl-1-bicyclo(2,2,2)octyl)-2,3-dicyanophenyl]-2-(3-fluoro-4-propylphenyl)-ethan
1-[4-(4-Pentyl-1-bicyclo(2,2,2)octyl)-2,3-dicyanophenyl]-2-(3-fluoro-4-pentylphenyl)-ethan
1-[4-(4-Pentyl-1-bicyclo(2,2,2)octyl)-2,3-dicyanophenyl]-2-(4-butoxy-3-fluorophenyl)-ethan

1-[4-(4-Pentyl-1-bicyclo(2,2,2)octyl)-2,3-dicyanophenyl]-2-(3-fluoro-4-propanoyloxyphenyl)-ethan
1-[4-(4-Propyl-1-bicyclo(2,2,2)octyl)-2,3-dicyanophenyl]-2-(3-chloro-4-propylphenyl)-ethan
1-[4-(4-Propyl-1-bicyclo(2,2,2)octyl)-2,3-dicyanophenyl]-2-(3-bromo-4-pentylphenyl)-ethan
1-[4-(4-Propyl-1-bicyclo(2,2,2)octyl)-2,3-dicyanophenyl]-2-(4-butoxy-3-chlorophenyl)-ethan
1-[4-(4-Propyl-1-bicyclo(2,2,2)octyl)-2,3-dicyanophenyl]-2-(3-chloro-4-propanoyloxyphenyl)-ethan
1-[-(4-Pentyl-1-bicyclo(2,2,2)octyl)-2,3-dicyanophenyl]-2-(3-chloro-4-propylphenyl)-ethan
1-[4-(4-pentyl-1-bicyclo(2,2,2)octyl)-2,3-dicyanophenyl]-2-(3-chloro-4-pentylphenyl)-ethan
1-[4-(4-Pentyl-1-bicyclo(2,2,2)octyl)-2,3-dicyanophenyl]-2-(4-butoxy-3-chlorophenyl)-ethan
1-[4-(4-Pentyl-1-bicyclo(2,2,2)octyl)-2,3-dicyanophenyl]-2-(3-chloro-4-propanoyloxyphenyl)-ethan
1-[4-(4-Propyl-1-bicyclo(2,2,2)octyl)-2,3-dicyanophenyl]-2-(3-bromo-4-propylphenyl)-ethan
1-[4-(4-Propyl-1-bicyclo(2,2,2)octyl)-2,3-dicyanophenyl]-2-(3-bromo-4-pentylphenyl)-ethan
1-[4-(4-Propyl-1-bicyclo(2,2,2)octyl)-2,3-dicyanophenyl]-2-(3-bromo-4-butoxyphenyl)-ethan
1-[4-(4-Propyl-1-bicyclo(2,2,2)octyl)-2,3-dicyanophenyl]-2-(3-bromo-4-propanoyloxyphenyl)-ethan
1-[4-(4-Pentyl-1-bicyclo(2,2,2)octyl)-2,3-dicyanophenyl]-2-(3-bromo-4-propylphenyl)-ethan
1-[4-(4-Pentyl-1-bicyclo(2,2,2)octyl)-2,3-dicyanophenyl]-2-(3-bromo-4-pentylphenyl)-ethan
1-[4-(4-Pentyl-1-bicyclo(2,2,2)octyl)-2,3-dicyanophenyl] 2 (3-bromo-4-butoxyphenyl)-ethan
1-[4-(4-Pentyl-1-bicyclo(2,2,2)octyl)-2,3-dicyanophenyl]-2-(3-bromo-4-propanoyloxyphenyl)-ethan
1-[4-(4-Propyl-1-bicyclo(2,2,2)octyl)-2,3-dicyanophenyl]-2-(3-cyano-4-propylphenyl)-ethan
1-[(4-(4-Propyl-1-bicyclo(2,2,2)octyl)-2,3-dicyanophenyl]-2-(3-cyano-4-pentylphenyl)-ethan
1-[4-(4-Propyl-1-bicyclo(2,2,2)octyl)-2,3-dicyanophenyl]-2-(4-butoxy-3-cyanophenyl)-ethan
1-[4-(4-Propyl-1-bicyclo(2,2,2)octyl)-2,3-dicyanophenyl]-2-(3-cyano-4-propanoyloxyphenyl)-ethan
1-[4-(4-Pentyl-1-bicyclo(2,2,2)octyl)-2,3-dicyanophenyl]-2-(3-cyano-4-propylphenyl)-ethan
1-[4-(4-Pentyl-1-bicyclo(2,2,2)octyl)-2,3-dicyanophenyl]-2-(3-cyano-4-pentylphenyl)-ethan
1-[4-(4-Pentyl-1-bicyclo(2,2,2)octyl)-2,3-dicyanophenyl]-2-(4-butoxy-3-cyanophenyl)-ethan
1-[4-(4-Pentyl-1-bicyclo(2,2,2)octyl)-2,3-dicyanophenyl]-2-(3-cyano-4-propanoyloxyphenyl)-ethan
1-[4-(4-Propyl-1-bicyclo(2,2,2)octyl)-2,3-dicyanophenyl]-2-(2-fluoro-4-propylphenyl)-ethan
1-[4-(4-Propyl-1-bicyclo(2,2,2)octyl)-2,3-dicyanophenyl]-2-(2-fluoro-4-pentylphenyl)-ethan
1-[4-(4-Propyl-1-bicyclo(2,2,2)octyl)-2,3-dicyanophenyl]-2-(4-butoxy-2-fluorophenyl)-ethan
1-[4-(4-Propyl-1-bicyclo(2,2,2)octyl)-2,3-dicyanophenyl]-2-(2-fluoro-4-propanoyloxyphenyl)-ethan
1-[4-(4-Pentyl-1-bicyclo(2,2,2)octyl)-2,3-dicyanophenyl]-2-(2-fluoro-4-propylphenyl)-ethan
1-[4-(4-Pentyl-1-bicyclo(2,2,2)octyl)-2,3-dicyanophenyl]-2-(2-fluoro-4-pentylphenyl)-ethan
1-[4-(4-Pentyl-1-bicyclo(2,2,2)octyl)-2,3-dicyanophenyl]-2-(4-butoxy-2-fluorophenyl)-ethan
1-[4-(4-Pentyl-1-bicyclo(2,2,2)octyl)-2,3-dicyanophenyl]-2-(2-fluoro-4-propanoyloxyphenyl)-ethan
1-[4-(4-Propyl-1-bicyclo(2,2,2)octyl)-2,3-dicyanophenyl]-2-(2-chloro-4-propylphenyl)-ethan
1-[4-(4-Propyl-1-bicyclo(2,2,2)octyl)-2,3-dicyanophenyl]-2-(2-chloro-4-pentylphenyl)-ethan
1-[4-(4-propyl-1-bicyclo(2,2,2)octyl)-2,3-dicyanophenyl]-2-(2-butoxy-2-chlorophenyl)-ethan
1-[4-(4-Propyl-1-bicyclo(2,2,2)-octyl)-2,3-dicyanophenyl]-2-(2-chloro-4-propanoyloxvphenyl)-ethan
1-[4-(4-Pentyl-1-bicyclo(2,2,2)octyl)-2,3-dicyanophenyl]-2-(2-chloro-4-propylphenyl)-ethan
1-[4-(4-pentyl-1-bicyclo(2,2,2)octyl)-2,3-dicyanophenyl]-2-(2-chloro-4-pentylphenyl)-ethan
1-[4-(4-Pentyl-1-bicyclo(2,2,2)octyl)-2,3-dicyanophenyl]-2-(2-butoxy-2-chlorophenyl)-ethan
1-[4-(4-Pentyl-1bicyclo(2,2,2)octyl)-2,3-dicyanophenyl]-2-(2-chloro-4-propanoyloxyphenyl)-ethan
1-[4-(4-Propyl-1-bicyclo(2,2,2)octyl)-2,3-dicyanophenyl]-2-(2-bromo-4-propylphenyl)-ethan
1-[4-(4-Propyl-1-bicyclo(2,2,2)octyl)-2,3-dicyanophenyl]-2-(2-bromo-4-pentylphenyl)-ethan
1-[4-(4-Propyl-1-bicyclo(2,2,2)octyl)-2,3-dicyanophenyl]-2-(2-bromo-4-butoxyphenyl)-ethan
1-[4-(4-Propyl-1-bicyclo(2,2,2)octyl)-2,3-dicyanophenyl]-1-(2-bromo-4-propanoyloxyphenyl)-ethan
1-[4-(4-Pentyl-1-bicyclo(2,2,2)octyl)-2,3-dicyanophenyl]-2-(2-bromo-4-propylphenyl)-ethan
1-[4-(4-Pentyl-1-bicyclo(2,2,2)octyl)-2,3-dicyanophenyl]-2-(2-bromo-4-pentylphenyl)-ethan
1-[4-(4-Pentyl-1-bicyclo(2,2,2)octyl)-2,3-dicyanophenyl]-2-(2-bromo-4-butoxyphenyl)-ethan
1-[4-(4-Pentyl-1-bicyclo(2,2,2)octyl)-2,3-dicyanophenyl]-1-(2-bromo-4-propanoyloxyphenyl)-ethan
1-[4-(4-Propyl-1-bicyclo(2,2,2)octyl)-2,3-dicyanophenyl]-2-(2-cyano-4-propylphenyl)-ethan
1-[4-(4-Propyl-1-bicyclo(2,2,2)octyl)-2,3-dicyanophenyl]-2-(2-cyano-4-pentylphenyl)-ethan
1-[4-(4-Propyl-1-bicyclo(2,2,2)octyl)-2,3-dicyanophenyl]-2-(4-butoxy-2-cyanophenyl)-ethan
1-[4-(4-Propyl-1-bicyclo(2,2,2)octyl)-2,3-dicyanophenyl]-2-(2-cyano-4-propanoyloxyphenyl)-ethan
1-[4-(4-Pentyl-1-bicyclo(2,2,2)octyl)-2,3-dicyanophenyl]-2-(2-cyano-4-propylphenyl)-ethan
1-[4-(4-Pentyl-1-bicyclo(2,2,2)octyl)-2,3-dicyanophenyl]-2-(2-cyano-4-pentylphenyl)-ethan
1-[4-(4-Pentyl-1-bicyclo(2,2,2)octyl)-2,3-dicyanophenyl]-2-(4-butoxy-2-cyanophenyl)-ethan
1-[4-(4-Pentyl-1-bicyclo(2,2,2)octyl)-2,3-dicyanophenyl]-2-(2-cyano-4-propanoyloxyphenyl)-ethan
1-(4-Propyl-1-bicyclo(2,2,2)octyl)-2,3-dicyano-4-(trans-4-propylyclohexyl)-benzol
1-(4-Propyl-1-bicyclo(2,2,2)octyl)-2,3-dicyano-4-(trans-4-pentylcyclohexyl)-benzol
1-(4-Pentyl-1-bicyclo(2,2,2)octyl)-2,3-dicyano-4-(trans-4-propylcyclohexyl)-benzol
1-(4-Pentyl-1-bicyclo(2,2,2)octyl)-2,3-dicyano-4-(trans-4-pentylcyclohexyl)-benzol
1-[4-(4-Propyl-1-bicyclo(2,2,2)octyl)-2,3-dicyanophenyl]-2-(trans-4-propylcyclohexyl)-ethan
1-[4-(4-Propyl-1-bicyclo(2,2,2)octyl)-2,3-dicyanophenyl]-2-(trans-4-pentylcyclohexyl)-ethan
1-[4-(4-Pentyl-1-bicyclo(2,2,2)octyl)-2,3-dicyanophenyl]-2-(trans-4-propylcyclohexyl)-ethan
1-[4-(4-Pentyl-1-bicyclo(2,2,2)octyl)-2,3-dicyanophenyl]-2-(trans-4-pentylcyclohexyl)-ethan

4-(4-Propyl-1-bicyclo(2,2,2)octyl)-2,3-dicyanophenyl-trans-4-propylcyclohexan-1-carboxylat
4-(4-Propyl-1-bicyclo(2,2,2)octyl)-2,3-dicyanophenyl-trans-4-pentylcyclohexan-1-carboxylat
4-(4-Pentyl-1-bicyclo(2,2,2)octyl)-2,3-dicyanophenyl-trans-4-propylcyclohexan-1-carboxylat
4-(4-Pentyl-1-bicyclo(2,2,2)octyl)-2,3-dicyanophenyl-trans-4-pentylcyclohexan-1-carboxylat
1,4-Bis(4-propyl-1-bicyclo(2,2,2)octyl)-2,3-dicyano-benzol
1,4-Bis(4-pentyl-1-bicyclo(2,2,2)octyl)-2,3-dicyano-benzol
1-(4-Pentyl-1-bicyclo(2,2,2)octyl)-4-(4-propyl-1-bicyclo-(2,2,2)octyl)-2,3-dicyano-benzol
1-[4-(4-Propyl-1-bicyclo(2,2,2)octyl)-2,3-dicyanophenyl]-2-(4-propyl-1-bicyclo(2,2,2)octyl)-ethan
1-[4-(4-Propyl-1-bicyclo(2,2,2)octyl)-2,3-dicyanophenyl]-2-(4-pentyl-1-bicyclo(2,2,2)octyl)-ethan
1-[4-(4-Pentyl-1-bicyclo(2,2,2)octyl)-2,3-dicyanophenyl]-2-(4-propyl-1-bicyclo(2,2,2)octyl)-ethan
1-[4-(4-Pentyl-1-bicyclo(2,2,2)octyl)-2,3-dicyanophenyl]-2-(4-pentyl-1-bicyclo(2,2,2)octyl)-ethan
4-(4-Propyl-1-bicyclo(2,2,2)-octyl)-2,3-dicyanophenyl-4-pro-pylbicyclo(2,2,2)octan-1-carboxylat
4-(4-Propyl-1-bicyclo(2,2,2)octyl)-2,3-dicyanophenyl-4-pen-tylbicyclo(2,2,2)octan-1-carboxylat
4-(4-Pentyl-1-bicyclo(2,2,2)octyl)-2,3-dicyanophenyl-4-pro-pylbicyclo(2,2,2)octan-1-carboxylat
4-(4-Pentyl-1-bicyclo(2,2,2)octyl)-2,3-dicyanophenyl-4-pen-tylbicyclo(2,2,2)octan-1-carboxylat
1-(4-Propyl-1-bicyclo(2,2,2)octyl)-2,3-dicyano-4-(4-propyl-1-bicyclo(2,2,2)octyl)-methoxy-benzol
1-(4-Propyl-1-bicyclo(2,2,2)octyl)-2,3-dicyano-4-(4-pentyl-1-bicyclo(2,2,2)octyl)-methoxy-benzol
1-(4-Pentyl-1-bicyclo(2,2,2)octyl)-2,3-dicyano-4-(4-propyl-1-bicyclo(2,2,2)octyl)-methoxy-benzol
1-(4-Pentyl-1-bicyclo(2,2,2)octyl)-2,3-dicyano-4-(4-pentyl-1-bicyclo(2,2,2)octyl)-methoxy-benzol
4-(4-Propyl-1-bicyclo(2,2,2)octyl)-methoxy-2,3-dicyano-4'-propyl-biphenyl
4-(4-Propyl-1-bcyclo(2,2,2)octyl)-methoxy-2,3-dicyano-4'-pentyl-biphenyl
4-(4-Propyl-1-bicyclo(2,2,2)octyl)-methoxy-2,3-dicyano-4'-butoxy-biphenyl
4-(4-Propyl-1-bicyclo(2,2,2)octyl)-methoxy-2,3-dicyano-4'-propanoyloxy-biphenyl
4-(4-Pentyl-1-bicyclo(2,2,2)octyl)-methoxy-2,3-dicyano-4'-propyl-biphenyl
4-(4-Pentyl-1-bicyclo(2,2,2)octyl)-methoxy-2,3-dicyano-4'-butoxy-biphenyl
4-(4-Pentyl-1-bicyclo(2,2,2)octyl)-methoxy-2,3-dicyano-4'-propanoyloxy-biphenyl
4-[2-(4-Propyl-1-bicyclo(2,2,2)octyl)-ethyl]-2,3-dicyano-4'-propyl-biphenyl
4-[2-(4-Propyl-1-bicyclo(2,2,2)octyl)-ethyl]-2,3-dicyano-4'-pentyl-biphenyl
4-[2-(4-Propyl-1-b cyclo(2,2,2)octyl)-ethyl]-2,3-dicyano-4'-butoxy-biphenyl
4-[2-(4-Propyl-1-bicyclo(2,2,2)octyl)-ethyl]-2,3-dicyano-4'-propanoyloxy-biphenyl
4-[2-(4-Pentyl-1-bicyclo(2,2,2)octyl)-ethyl]-2,3-dicyano-4'-propyl-biphenyl
4-[2-(4-Pentyl-1-bicyclo(2,2,2)octyl)-ethyl]-2,3-dicyano-4'-pentyl-biphenyl
4-[2-(4-Pentyl-1-bicyclo(2,2,2)octyl)-ethyl]-2,3-dicyano-4'-butoxy-biphenyl
4-[2-(4-Pentyl-1-bicyclo(2,2,2)octyl)-ethyl]-2,3-dicyano-4'-propanoyloxy-biphenyl
4-(2,3-Dicyano-4'-propyl-biphenylyl)-4-propyl-bicyclo(2,2,2)-octan-1-carboxylat
4-(2,3-Dicyano-4'-pentyl-biphenylyl)-4-propyl-bicyclo(2,2,2)-octan-1-carboxylat
4-(4'-Butoxy-2,3-dicyano-biphenylyl)-4-propyl-bicyclo(2,2,2)-octan-1-carboxylat
4-(4'-Butanoyloxy-2,3-dicyano-biphenylyl)-4-propyl-bicyclo-(2,2,2)octan-1-carboxylat
4-(2,3-Dicyano-4'-propyl-biphenylyl)-4-pentyl-bicyclo(2,2,2) octan-1-carboxylat
4-(2,3-Dicyano-4'-pentyl-biphenylyl)-4-pentyl-bicyclo(2,2,2)-octan-1-carboxylat
4-(4'-Butoxy-2,3-dicyano-biphenylyl)-4-pentyl-bicyclo(2,2,2)-octan-1-carboxylat
4-(4'-Butanoyloxy-2,3-dicyano-biphenylyl)-4-pentyl-bicyclo-(2,2,2)octan-1-carboxylat
1-(4-Propyl-1-bicyclo(2,2,2)octyl)-methoxy-2,3-dicyano-4-(trans-4-propylcyclohexyl)-benzol
1-(4-Propyl-1-bicyclo(2,2,2)octyl)-methoxy-2,3-dicyano-4-(trans-4-pentylcyclohexyl)-benzol
1-(4-Pentyl-1-bicyclo(2,2,2)octyl)-methoxy-2,3-dicyano-4-(trans-4-propylcyclohexyl)-benzol
1-(4-Pentyl-1-bicyclo(2,2,2)octyl)-methoxy-2,3-dicyano-4-(trans-4-pentylcyclohexyl)-benzol
2,3-Dicyano-1-(trans-4-propylcyclohexyl)-4-(2-(4-propyl-1-bicyclo(2,2,2)octyl)-ethyl-benzol
2,3-Dicyano-1-(trans-4-pentylcyclohexyl)-4-(2-(4-propyl-1-bicyclo(2,2,2)octyl)-ethyl-benzol
2,3-Dicyano-1-(trans-4-propylcyclohexyl)-4-(2-(4-pentyl-1-bicyclo(2,2,2)octyl)-ethyl-benzol
2,3-Dicyano-1-(trans-4-pentylcyclohexyl)-4-(2-(4-pentyl-1-bicyclo(2,2,2)octyl)-ethyl-benzol
1-[2,3-Dicyano-4-(trans-4-Propylcyclohexyl)]-phenyl-4-propyl-bicyclo(2,2,2)octan-1-carboxylat
1-[2,3-Dicyano-4-(trans-4-pentylcyclohexyl)]-phenyl-4-propyl-bicyclo(2,2,2)octan-1-carboxylat
1-[2,3-Dicyano-4-(trans-4-propylcyclohexyl)]-phenyl-4-pentyl-bicyclo(2,2,2)octan-1-carboxylat
1-[2,3-Dicyano-4-(trans-4-pentylcyclohexyl)]-phenyl-4-pentyl-bicyclo(2,2,2)octan-1-carboxylat

**Patentansprüche**

1. Anisotrope Verbindungen mit negativer DK-Anisotropie und verbesserter fotochemischer Beständigkeit, gekennzeichnet durch die Formel (1)

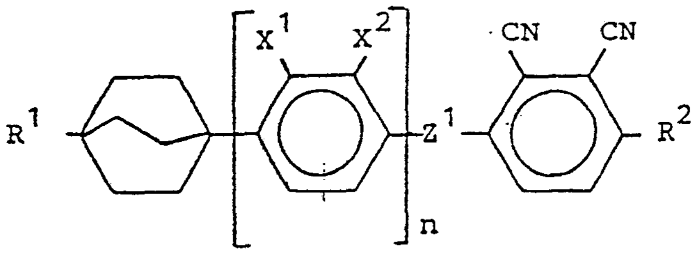

(1)

in der n Null oder Eins ist, $Z^1$ die Kovalenzbindung oder eine Brückengruppe der Formeln $-CH_2O-$, $-CH_2CH_2-$ oder -COO ist, $X^1$ und $X^2$ gleich oder verschieden und Wasserstoffoder Halogenatome oder Nitrilgruppen darstellen, $R^1$ und $R^2$ Wasserstoffatome oder Alkyl-, Alkoxy-, Alkanoyloxyoder Alkoxycarbonylgruppen mit jeweils 1 bis 12 C-Atomen im Alkylteil in gerader oder verzweigter und gegebenenfalls chiraler Kette bedeuten, wenn n gleich Null oder Eins ist, und wobei entweder $R^1$ oder $R^2$ einen Rest der Formel (1a), (1b) oder (1c)

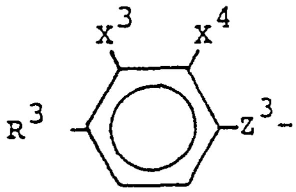

(1a)

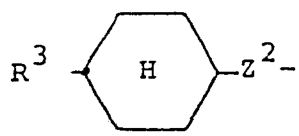

(1b)

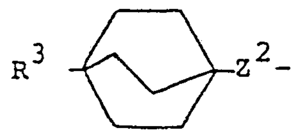

(1c)

bedeuten kann, wenn n Null ist, wobei $Z^2$ die für $Z^1$ angegebene Bedeutung hat, $Z^3$ die Kovalenzbindung oder eine Brückengruppe der Formeln $-CH_2CH_2-$ oder -COO- ist, $R^3$ das Wasserstoffatom oder eine Alkyl-, Alkoxy-, Alkanoyloxygruppe oder Alkoxycarbonylgruppe mit 1 bis 12 C-Atomen im Alkylteil in gerader oder verzweigter und gegebenenfalls chiraler Kette ist, und $X^3$, $X^4$ eine der für $X^1$, $X^2$ angegebenen Bedeutungen haben, mit der Massgabe, dass keiner der im Molekül vorhandenen Benzolringe gleichzeitig direkt mit zwei Sauerstoffatomen und direkt mit Nitrilgruppen verbunden ist.

2. Verbindung nach Patentanspruch 1, gekennzeichnet durch die Formel (2)

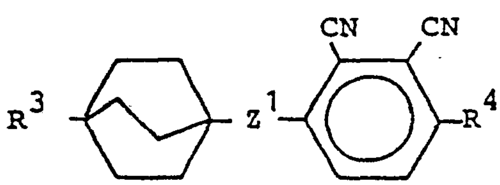

(2)

in welcher $R^3$ und $Z^1$ die in Patentanspruch 1 angegebene Bedeutung haben und $R^4$ eine der für $R^3$ genannten Bedeutungen hat.

3. Verbindung nach Patentanspruch 1, gekennzeichnet durch die Formel (3)

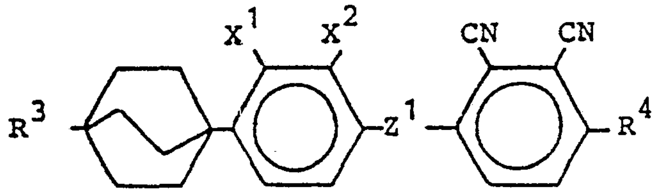

(3)

in welcher $X^1$, $X^2$, $Z^1$ und $R^3$ die in Patentanspruch 1 angegebene Bedeutung haben und R $^4$ eine der für $R^3$ genannten Bedeutungen hat.

4. Verbindung nach Patentanspruch 1, gekennzeichnet durch die Formel (4)

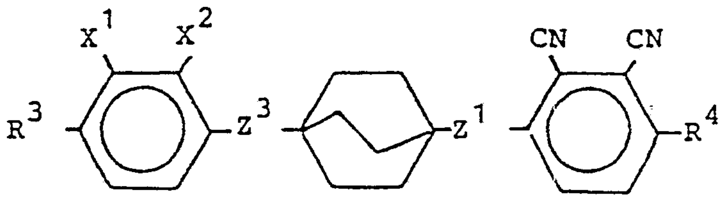

(4)

in der $X^1$, $X^2$, $Z^1$, $Z^3$ und $R^3$ die in Patentaspruch 1 angegebenen Bedeutungen haben und $R^4$ eine der für $R^3$ genannten Bedeutungen hat.

5. Verbindung nach Patentanspruch 1, gekennzeichnet durch die Formel (5)

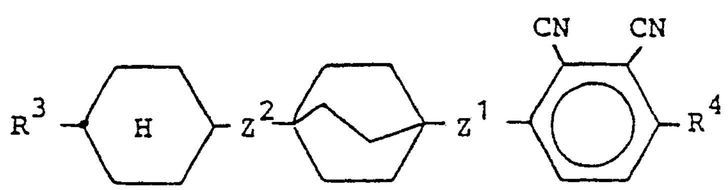

(trans)

(5)

in der $Z^1$, $Z^2$ und $R^3$ die in Patentanspruch 1 angegebenen Bedeutungen haben und $R^4$ eine der für $R^3$ genannten Bedeutungen hat.

6. Verbindung nach Patentanspruch 1, gekennzeichnet durch die Formel (6)

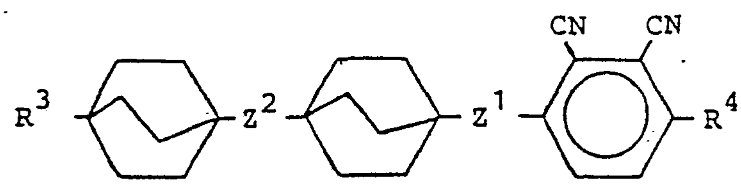

(6)

in der $Z^1$, $Z^2$ und $R^3$ die in Patentanspruch 1 angegebenen Bedeutungen haben und $R^4$ eine der für $R^3$ genannten Bedeutungen hat.

7. Verbindung nach Patentanspruch 1, gekennzeichnet durch die Formel (7)

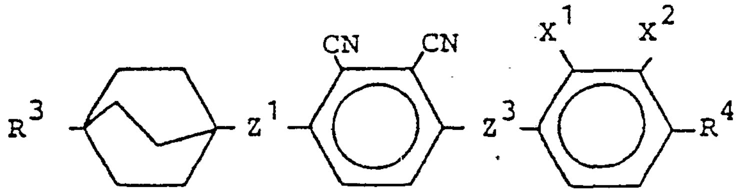 (7)

in der $Z^1$, $Z^3$, $X^1$, $X^2$ und $R^3$ die in Patentanspruch 1 angegebenen Bedeutungen haben und $R^4$ eine der für $R^3$ genannten Bedeutungen hat.

8. Verbindung nach Patentanspruch 1, gekennzeichnet durch die Formel (8)

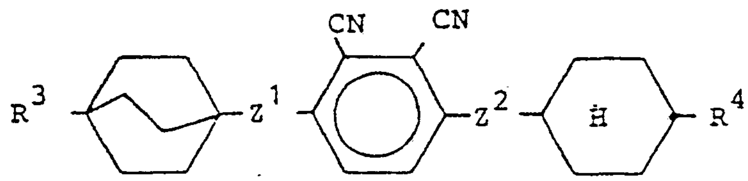 (8)

(trans)

in der $Z^1$, $Z^2$ und $R^3$ die in Patentanspruch 1 angegebenen Bedeutungen haben und $R^4$ eine der für $R^3$ genannten Bedeutungen hat.

9. Verbindung nach Patentanspruch 1, gekennzeichnet durch die Formel (9)

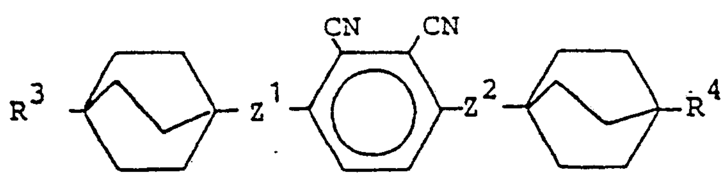 (9)

in der $Z^1$, $Z^2$ und $R^3$ die in Patentanspruch 1 angegebenen Bedeutungen haben und $R^4$ eine der für $R^3$ genannten Bedeutungen hat.

10. Flüssigkristallmischung, dadurch gekennzeichnet, dass sie mindestens eine anisotrope Verbindung gemäss Patentanspruch 1 enthält.

11. Mischung nach Patentanspruch 10, dadurch gekennzeichnet, dass sie mindestens eine Verbindung gemäss einem der Patentansprüche 2 bis 9 enthält.

**Claims:**

1. An anisotropic compound having a negative DC anisotropy and improved photochemical stability, of the formula (1)

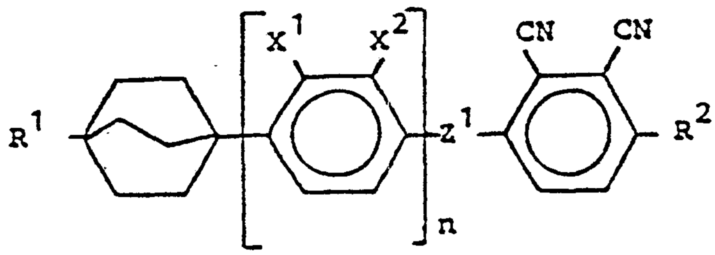

(1)

in which n is zero or one, $Z^1$ is the covalent bond or a bridge group of the formula $-CH_2O-$, $-CH_2CH_2-$ or $-COO-$, $X^1$ and $X^2$ are identical or different and are hydrogen or halogen atoms or nitrile groups, and, if n is zero or one, $R^1$ and $R^2$ are hydrogen atoms or alkyl, alkoxy, alkanoyloxy or alkoxycarbonyl groups with in each case 1 to 12 carbon atoms in the alkyl part in a straight or branched chain, which may be chiral, and, if n is zero, either $R^1$ or $R^2$ can be a radical of the formula (1a), (1b) or (1c)

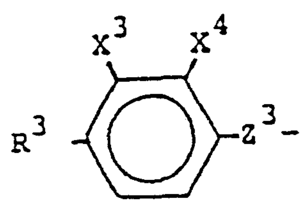

(1a)

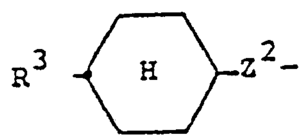

(1b)

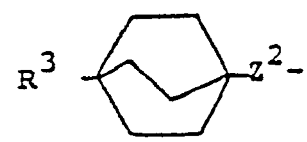

(1c)

in which $Z^2$ has the meanings given for $Z^1$, $Z^3$ is the covalent bond or a bridge group of the formulae $-CH_2CH_2-$ or $-COO-$ and $R^3$ is a hydrogen atom or an alkyl, alkoxy, alkanoyloxy or alkoxycarbonyl group with 1 to 12 carbon atoms in the alkyl part in a straight or branched chain, which may be chiral, and $X^3$ and $X^4$ have one of the meanings given for $X^1$ and $X^2$, with the proviso that none of the benzene rings present in the molecule are at the same time bonded directly to two oxygen atoms and directly to nitrile groups.

2. A compound as claimed in patent claim 1, of the formula (2)

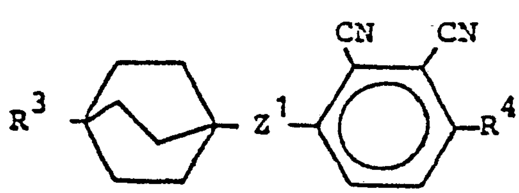

(2)

in which $R^3$ and $Z^1$ have the meanings given in patent claim 1 and $R^4$ has one of the meanings given for $R^3$

3. A compound as claimed in patent claim 1, of the formula (3)

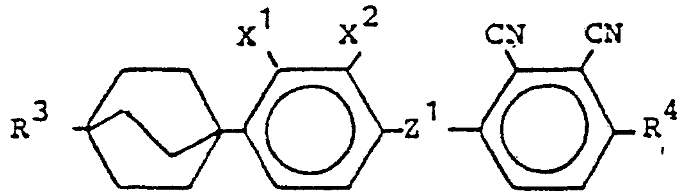

(3)

in which $X^1$, $X^2$, $Z^1$ and $R^3$ have the meanings given in patent claim 1 and $R^4$ has one of the meanings given for $R^3$.

4. A compound as claimed in patent claim 1, of the formula (4)

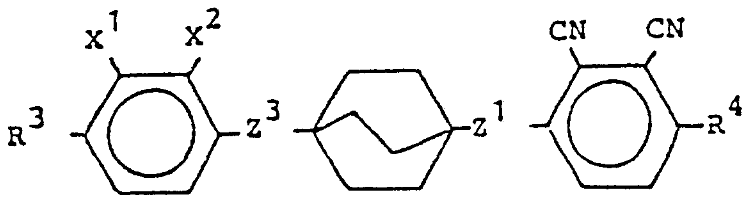

(4)

in which $X^1$, $X^2$, $Z^1$, $Z^3$ and $R^3$ have the meanings given ih Patent claim 1 and $R^C$ has one of the meanings given for $R^3$.

5. A compound as claimed in patent claim 1, of the formula (5)

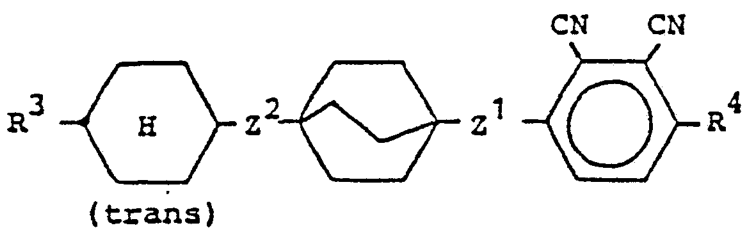

(5)

in which $Z^1$, $Z^2$ and $R^3$ hava the meanings given in patent claim 1 and $R^4$ has one of the meanings given for $R^3$.

6. A compound as claimed in patent claim 1, of the formula (6)

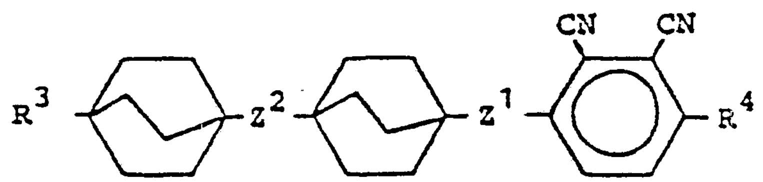

(6)

in which $Z^1$, $Z^2$ and $Z^3$ have the meanings given in patent claim 1 and $R^4$ has one of the meanings given for $R^3$.

7. A compound as claimed in patent claim 1, of the formula (7)

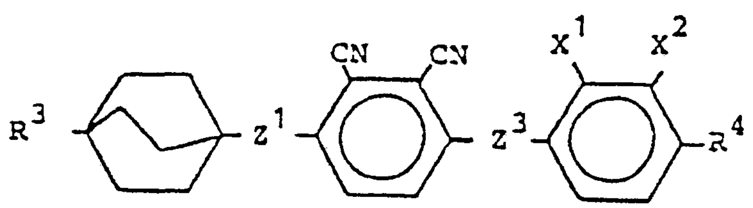

(7)

in which $Z^1$, $Z^3$, $X^1$, $X^2$ and $R^3$ have the meanings given in patent claim 1 and $R^4$ has one of the meanings given for $R^3$.

8. A compound as claimed in patent claim 1, of the formula (8)

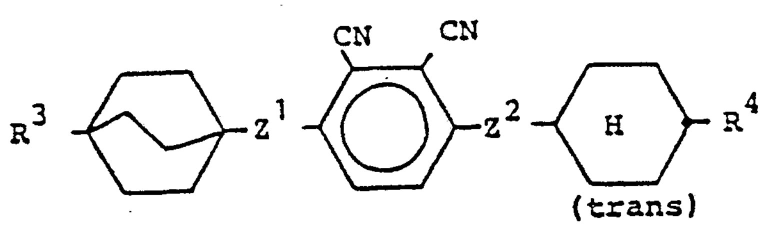

(8)

in which $Z^1$, $Z^2$ and $R^3$ have the meanings given in patent claim 1 and $R^4$ has one of the meanings given for $R^3$.

9. A compound as claimed in patent claim 1, of the formula (9)

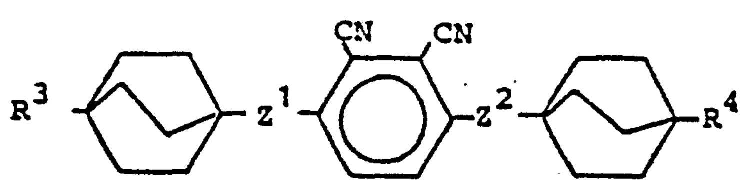

(9)

in which $Z^1$, $Z^2$ and $R^3$ have the meanings given in patent claim 1 and $R^4$ has one of the meanings given for $R^3$.

10. A liquid crystal mixture which contains at least one anisotropic compound as claimed in patent claim 1.

11. A mixture as claimed in patent claim 10, which contains at least one compound as claimed in any one of patent claims 2 to 9.

**Revendications**

1 - Composés anisotropes à anisotropie diélectrique négative et stabilité photochimique améliorée, caractérisés en ce qu'ils lèpondent à la formule 1

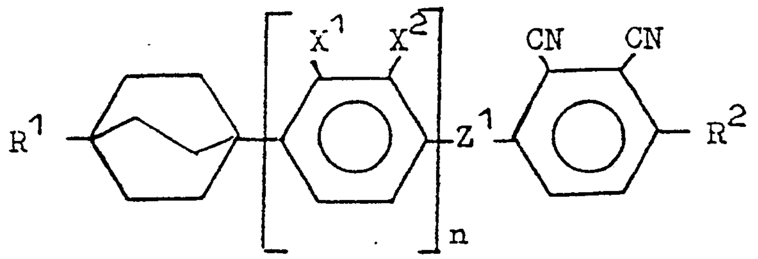
(1)

dans laquelle n est égal à 0 ou 1, $Z^1$ représente la liaison covalente ou un pont de formule $-CH_2O-$, $-CH_2CH_2-$ou $-COO-$, $X^1$ et $X^2$, ayant des significations identiques ou différentes, représentent des atomes d'hydrogène ou d'halogènes ou des groupes nitrile, $R^1$ et $R^2$ représentent des atomes d'hydrogène ou des groupes alkyle, alcoxy, alcanoyloxy ou alcoxycarbonyle contenant chacun 1 à 12 atomes de carbone dans la partie alkyle en chaîne droite ou ramifiée et éventuellement chirale lorsque n est égal à 0 ou 1, $R^1$ ou $R^2$ peut représenter un reste de formule 1a, 1b ou 1c

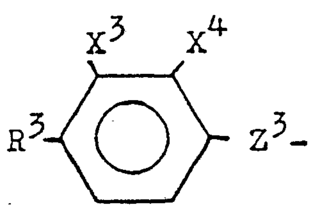
(1a)

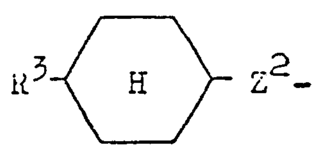
(1b)

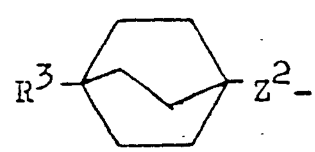
(1c) .

lorsque n est ègal à 0, $Z^2$ a les significations indiquées pour $Z^1$, $Z^3$ représente une liaison covalente ou un pont de formule $-CH_2CH_2-$ ou $-COO-$, $R^3$ représente un atome d'hydrogène ou un groupe alkyle alcoxy, alcanoyloxy ou alcoxycarbonyle contenant 1 à 12 atomes de carbone dans la partie alkyle en chaîne droite ou ramifiée et éventuellement chirale, et $X^3$, $X^4$ ont l'une des significations indiquées pour $X^1$, $X^2$, étant spécifiée et des noyaux benzéniques existant dans la molécule n'est relié simultanément directement avec deux atomes d'oxygène et directement avec des groupes nitrile.

2 - Composé selon la revendication 1, caractérisé en ce qu'il répond à la formule 2

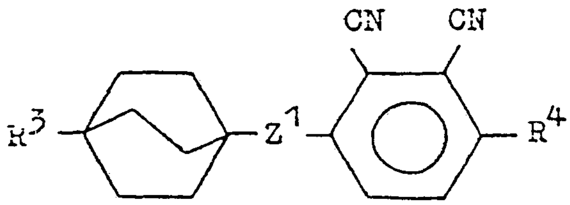

(2)

dans laquelle $R^3$ et $Z^1$ ont les significations indiquées dans la revendication 1 et $R^4$ a l'une des significations indiquées pour $R^3$.

3 - Composé selon la revendication 1, caractérisé en ce qu'il répond à la formule 3

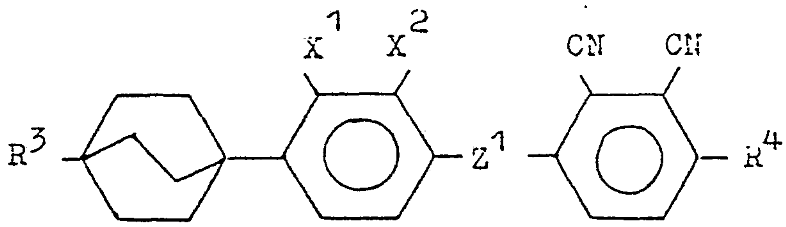

(3)

dans laquelle $X^1$, $X^2$, $Z^1$ et $R^3$ ont les significations indiquées dans la revendication 1 et $R^4$ a l'une des significations indiquées pour $R^3$.

4 - Composé selon la revendication 1, caractérisé en ce qu'il répond à la formule 4

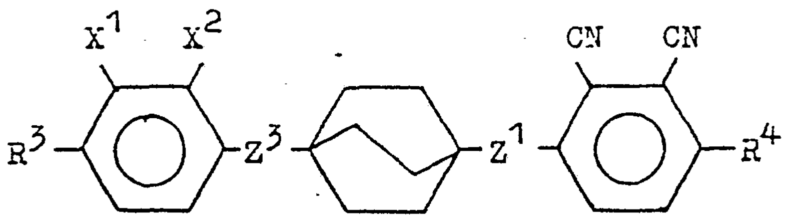

(4)

dans laquelle $X^1$, $X^2$, $Z^1$, $Z^3$ et $R^3$ ont les significations indiquées dans la revendication 1 et $R^4$ a l'une des significations indiquées pour $R^3$.

5 - Composé selon la revendication 1, caractérisé en ce qu'il répond à la formule 5

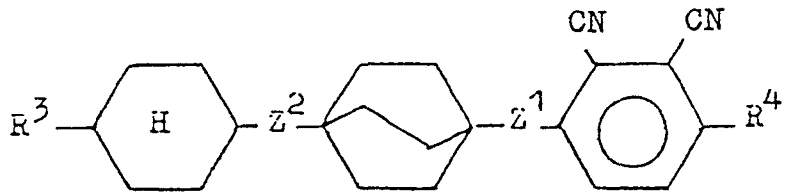 (5)

(trans)

dans laquelle $Z^1$, $Z^2$ et $R^3$ ont les significations indiquées dans la revendication 1 et $R^4$ a l'une des significations indiquées pour $R^3$.

6 - Composé selon la revendication 1, caractérisé en ce qu'il repond à la formule 6

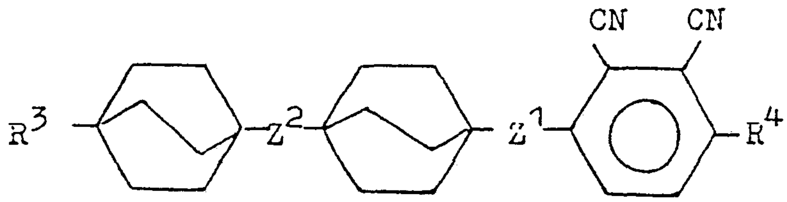 (6)

dans laquelle $Z^1$, $Z^2$ et $R^3$ ont les significations indiquées dans la revendication 1 et $R^4$ a l'une des significations indiquées pour $R^3$.

7 - Composé selon la revendication 1, caractérisé en ce qu'il répond à la formule 7

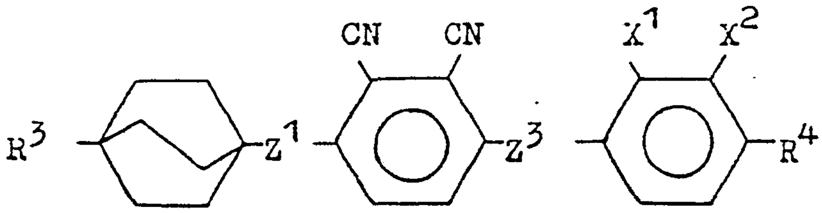 (7)

dans laquelle $Z^1$, $Z^3$, $X^1$, $X^2$ et $R^3$ ont les significations indiquées dans la revendication 1 et $R^4$ a l'une des significations indiquées pour $R^3$.

8 - Composé selon la revendication 1, caractérisé en ce qu'il répond à la formule 8

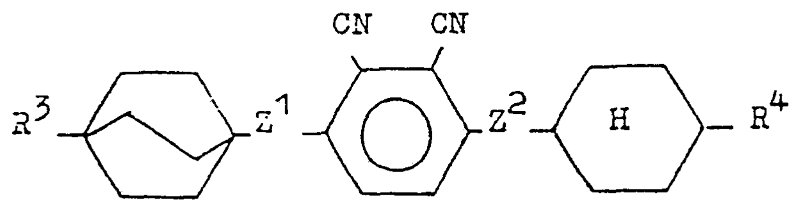

(8)

(trans)

dans laquelle $Z^1$, $Z^2$ et $R^3$ ont les significations indiquées dans la revendication 1 et $R^4$ a l'une des significations indiquées pour $R^3$.

9 - Composé selon la revendication 1, caractérisé en ce qu'il répond à la formule 9

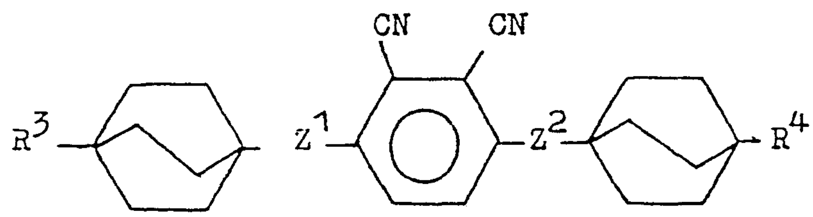

(9)

dans laquelle $Z^1$, $Z^2$ et $R^3$ ont les significations indiquées dans la revendication 1 et $R^4$ a l'une des significations indiquées pour $R^3$.

10 - Mélange à cristaux liquides, caractérisé en ce qu'il contient au moins un composé anisotrope selon la revendication 1.

11 - Mélange selon la revendication 10, caractérisé en ce qu'il contient au moins un composé selon l'une des revendications 2 à 9.